# EUROPEAN PATENT APPLICATION

(11) **EP 1 655 608 A1**
(43) Date of publication of application: **10.05.2006**
(21) Application number: 04292621.2
(22) Date of filing: 04.11.2004
(51) Int. Cl.: G01N 33/68

(54) **CFTR as a component of an autophagy signaling pathway, applications thereof to autophagic diseases, and determination of cystic fibrosis as an autophagic disease**

(71) Applicant: INSERM, 75654 Paris Cédex 13 (FR)
(72) Inventor: Mograbi, Baharia, 06300 Nice (FR); Corcelle, Elisabeth, 06240 Beausoleil (FR); Barriere, Hervé, 06100 Nice (FR); Poujeol, Philippe, 06670 Levens (FR)
(74) Representative: Paris, Fabienne

(57) **Abstract**

The present invention demonstrates that CFTR is a key factor of the autophagy signaling pathway. It further demonstrates that the kinase/CFTR pathways are essential to the maturation stage of autophagy.

The different aspects of the present invention involve CFTR, and more particularly the kinase/CFTR pathways, and their various uses and applications as a key component of the autophagy signaling pathway.

The present invention further identifies cystic fibrosis as an autophagic disease.

### Annex to the application documents - subsequently filed sequences listing

SEQUENCE LISTING

<110>  INSERM

<120>  CFTR as a component of an autophagy signaling pathway,
       applications thereof to autophagic diseases, and determination of
        cystic fibrosis as an autophagic disease

<130>  FP - CFTR autophagy Mograbi

<140>  EP04292621.2
<141>  2004-11-04

<160>  56

<170>  PatentIn version 3.3

<210>  1
<211>  20
<212>  PRT
<213>  Homo sapiens

<400>  1

Arg Lys Gly Tyr Arg Gln Arg Leu Glu Leu Ser Asp Ile Tyr Gln Ile
1               5                   10                  15

Pro Ser Val Asp
            20

<210>  2
<211>  7
<212>  PRT
<213>  Homo sapiens

<400>  2

Lys Val Phe Ile Phe Ser Gly
1               5

<210>  3
<211>  8
<212>  PRT
<213>  Homo sapiens

<400>  3

Gln Lys Val Phe Ile Phe Ser Gly
1               5

<210>  4
<211>  9
<212>  PRT
<213>  Homo sapiens

<400>  4

Gln Lys Val Phe Ile Phe Ser Gly Thr
1               5

<210>  5
<211>  10
<212>  PRT
<213>  Homo sapiens

<400>  5

Pro Gln Lys Val Phe Ile Phe Ser Gly Thr
1               5                   10

<210>  6
<211>  11
<212>  PRT
<213>  Homo sapiens

<400>  6

Pro Gln Lys Val Phe Ile Phe Ser Gly Thr Phe
1               5                   10

<210>  7
<211>  7
<212>  PRT
<213>  Homo sapiens

<400>  7

Met Gln Arg Ser Pro Leu Glu
1               5

<210>  8
<211>  8
<212>  PRT
<213>  Homo sapiens

<400>  8

Met Gln Arg Ser Pro Leu Glu Lys
1               5

<210>  9
<211>  9
<212>  PRT
<213>  Homo sapiens

<400>  9

Met Gln Arg Ser Pro Leu Glu Lys Ala
1               5

<210>  10
<211>  10

<212>  PRT
<213>  Homo sapiens

<400>  10

Met Gln Arg Ser Pro Leu Glu Lys Ala Ser
1               5                   10


<210>  11
<211>  11
<212>  PRT
<213>  Homo sapiens

<400>  11

Met Gln Arg Ser Pro Leu Glu Lys Ala Ser Val
1               5                   10


<210>  12
<211>  7
<212>  PRT
<213>  Homo sapiens

<400>  12

Ser Leu Leu Gly Thr Pro Val
1               5


<210>  13
<211>  8
<212>  PRT
<213>  Homo sapiens

<400>  13

Ser Leu Leu Gly Thr Pro Val Leu
1               5


<210>  14
<211>  9
<212>  PRT
<213>  Homo sapiens

<400>  14

Phe Ser Leu Leu Gly Thr Pro Val Leu
1               5


<210>  15
<211>  10
<212>  PRT
<213>  Homo sapiens

<400>  15

Phe Ser Leu Leu Gly Thr Pro Val Leu Lys

1                   5                   10


<210>  16
<211>  11
<212>  PRT
<213>  Homo sapiens

<400>  16

Phe Ser Leu Leu Gly Thr Pro Val Leu Lys Asp
1               5                   10


<210>  17
<211>  7
<212>  PRT
<213>  Homo sapiens

<400>  17

Tyr Leu Leu Asp Ser Pro Phe
1               5


<210>  18
<211>  8
<212>  PRT
<213>  Homo sapiens

<400>  18

Tyr Leu Leu Asp Ser Pro Phe Gly
1               5


<210>  19
<211>  9
<212>  PRT
<213>  Homo sapiens

<400>  19

Leu Tyr Leu Leu Asp Ser Pro Phe Gly
1               5


<210>  20
<211>  10
<212>  PRT
<213>  Homo sapiens

<400>  20

Leu Tyr Leu Leu Asp Ser Pro Phe Gly Tyr
1               5                   10


<210>  21
<211>  11
<212>  PRT

<213>  Homo sapiens

<400>  21

Leu Tyr Leu Leu Asp Ser Pro Phe Gly Tyr Leu
1               5                   10


<210>  22
<211>  7
<212>  PRT
<213>  Artificial

<220>
<223>  mutated form of CFTR peptide

<400>  22

Tyr Leu Ser Asp Ser Pro Phe
1               5


<210>  23
<211>  8
<212>  PRT
<213>  Artificial

<220>
<223>  mutated form of CFTR peptide

<400>  23

Tyr Leu Ser Asp Ser Pro Phe Gly
1               5


<210>  24
<211>  9
<212>  PRT
<213>  Artificial

<220>
<223>  mutated form of CFTR peptide

<400>  24

Leu Tyr Leu Ser Asp Ser Pro Phe Gly
1               5


<210>  25
<211>  10
<212>  PRT
<213>  Artificial

<220>
<223>  mutated form of CFTR peptide

<400>  25

Leu Tyr Leu Ser Asp Ser Pro Phe Gly Tyr

1                   5                        10


<210>  26
<211>  11
<212>  PRT
<213>  Artificial

<220>
<223>  mutated form of CFTR peptide

<400>  26

Leu Tyr Leu Ser Asp Ser Pro Phe Gly Tyr Leu
1               5                   10


<210>  27
<211>  7
<212>  PRT
<213>  Artificial

<220>
<223>  mutated form of CFTR peptide

<400>  27

Tyr Leu Leu Asn Ser Pro Phe
1               5


<210>  28
<211>  8
<212>  PRT
<213>  Artificial

<220>
<223>  mutated form of CFTR peptide

<400>  28

Tyr Leu Leu Asn Ser Pro Phe Gly
1               5


<210>  29
<211>  9
<212>  PRT
<213>  Artificial

<220>
<223>  mutated form of CFTR peptide

<400>  29

Leu Tyr Leu Leu Asn Ser Pro Phe Gly
1               5


<210>  30
<211>  10

<212>  PRT
<213>  Artificial

<220>
<223>  mutated form of CFTR peptide

<400>  30

Leu Tyr Leu Leu Asn Ser Pro Phe Gly Tyr
1               5                   10


<210>  31
<211>  11
<212>  PRT
<213>  Artificial

<220>
<223>  mutated form of CFTR peptide

<400>  31

Leu Tyr Leu Leu Asn Ser Pro Phe Gly Tyr Leu
1               5                   10


<210>  32
<211>  7
<212>  PRT
<213>  Artificial

<220>
<223>  mutated form of CFTR peptide

<400>  32

Tyr Leu Leu Asp Ser His Phe
1               5


<210>  33
<211>  8
<212>  PRT
<213>  Artificial

<220>
<223>  mutated form of CFTR peptide

<400>  33

Tyr Leu Leu Asp Ser His Phe Gly
1               5


<210>  34
<211>  9
<212>  PRT
<213>  Artificial

<220>
<223>  mutated form of CFTR peptide

<400>  34

Leu Tyr Leu Leu Asp Ser His Phe Gly
1               5


<210>  35
<211>  10
<212>  PRT
<213>  Artificial

<220>
<223>  mutated form of CFTR peptide

<400>  35

Leu Tyr Leu Leu Asp Ser His Phe Gly Tyr
1               5                   10


<210>  36
<211>  11
<212>  PRT
<213>  Artificial

<220>
<223>  mutated form of CFTR peptide

<400>  36

Leu Tyr Leu Leu Asp Ser His Phe Gly Tyr Leu
1               5                   10


<210>  37
<211>  7
<212>  PRT
<213>  Homo sapiens

<400>  37

Ile Val Gln Lys Thr Pro Leu
1               5


<210>  38
<211>  8
<212>  PRT
<213>  Homo sapiens

<400>  38

Ile Val Gln Lys Thr Pro Leu Gln
1               5


<210>  39
<211>  9
<212>  PRT
<213>  Homo sapiens

<400>  39

Ser Ile Val Gln Lys Thr Pro Leu Gln
1               5


<210>  40
<211>  10
<212>  PRT
<213>  Homo sapiens

<400>  40

Ser Ile Val Gln Lys Thr Pro Leu Gln Met
1               5                   10


<210>  41
<211>  11
<212>  PRT
<213>  Homo sapiens

<400>  41

Ser Ile Val Gln Lys Thr Pro Leu Gln Met Asn
1               5                   10


<210>  42
<211>  7
<212>  PRT
<213>  Homo sapiens

<400>  42

Ser Phe Ser Ile Ser Pro Gly
1               5


<210>  43
<211>  8
<212>  PRT
<213>  Homo sapiens

<400>  43

Ser Phe Ser Ile Ser Pro Gly Gln
1               5


<210>  44
<211>  9
<212>  PRT
<213>  Homo sapiens

<400>  44

Ile Ser Phe Ser Ile Ser Pro Gly Gln
1               5

<210>  45
<211>  10
<212>  PRT
<213>  Homo sapiens

<400>  45

Ile Ser Phe Ser Ile Ser Pro Gly Gln Arg
1               5                   10


<210>  46
<211>  11
<212>  PRT
<213>  Homo sapiens

<400>  46

Ile Ser Phe Ser Ile Ser Pro Gly Gln Arg Val
1               5                   10


<210>  47
<211>  7
<212>  PRT
<213>  Artificial

<220>
<223>  mutated form of CFTR peptide

<400>  47

Ser Phe Ser Val Ser Pro Gly
1               5


<210>  48
<211>  8
<212>  PRT
<213>  Artificial

<220>
<223>  mutated form of CFTR peptide

<400>  48

Ser Phe Ser Val Ser Pro Gly Gln
1               5


<210>  49
<211>  9
<212>  PRT
<213>  Artificial

<220>
<223>  mutated form of CFTR peptide

<400>  49

Ile Ser Phe Ser Val Ser Pro Gly Gln
1               5

<210>  50
<211>  10
<212>  PRT
<213>  Artificial

<220>
<223>  mutated form of CFTR peptide

<400>  50

Ile Ser Phe Ser Val Ser Pro Gly Gln Arg
1               5                   10

<210>  51
<211>  11
<212>  PRT
<213>  Artificial

<220>
<223>  mutated form of CFTR peptide

<400>  51

Ile Ser Phe Ser Val Ser Pro Gly Gln Arg Val
1               5                   10

<210>  52
<211>  7
<212>  PRT
<213>  Artificial

<220>
<223>  mutated form of CFTR peptide

<400>  52

Ser Phe Ser Ile Arg Pro Gly
1               5

<210>  53
<211>  8
<212>  PRT
<213>  Artificial

<220>
<223>  mutated form of CFTR peptide

<400>  53

Ser Phe Ser Ile Arg Pro Gly Gln
1               5

<210>  54

<211>  9
<212>  PRT
<213>  Artificial

<220>
<223>  mutated form of CFTR peptide

<400>  54

Ile Ser Phe Ser Ile Arg Pro Gly Gln
1               5


<210>  55
<211>  10
<212>  PRT
<213>  Artificial

<220>
<223>  mutated form of CFTR peptide

<400>  55

Ile Ser Phe Ser Ile Arg Pro Gly Gln Arg
1               5                   10


<210>  56
<211>  11
<212>  PRT
<213>  Artificial

<220>
<223>  mutated form of CFTR peptide

<400>  56

Ile Ser Phe Ser Ile Arg Pro Gly Gln Arg Val
1               5                   10

## Description

### FIELD OF THE INVENTION:

The present invention generally relates to autophagy, and to the biological and medical applications thereof.
It more particularly relates to the use of CFTR (Cystic Fibrosis Transmembrane conductance Regulator) as a component of an autophagy signaling pathway, to the biological and medical applications thereof, particularly in the field of autophagic diseases and conditions. The present invention further identifies for the first time that cystic fibrosis appears as an autophagic disease.

The different aspects of the present invention all involve CFTR, and more particularly the kinases/CFTR pathways, as well as their various uses and applications as a key component of the autophagy signaling pathway.

In the present patent application, reference is made to various scientific publications (*via* a number between brackets); the reference to which this number directs is given in the "bibliographic references" section, which is listed before the "claims" section.

### BACKGROUND OF THE INVENTION:

### Autophagy:

Eukaryotic cells achieve the degradation of their unneeded or damaged cytoplasmic components by a lysosomal process termed autophagy (for review, see refs (*1*, *2*).
The *initial* step of autophagy is the surrounding of cytoplasmic and organelle portions of the cell by a single isolation membrane (also known as phagophore). This sequestration results in the formation of a closed doubled-membrane structure containing undigested cytoplasmic materials including organelles. This double-membrane vacuole is termed autophagosome (or autophagic immature vacuole).

Next, during the *maturation* step, the outer membrane of the autophagosome fuses with the membrane of a lysosome, to become an autolysosome (also termed autophagolysosome). Lysosomal hydrolytic enzymes and acidification through vacuolar-type H+ ATPase (V-ATPase) activity result in the cytoplasm-derived contents being degraded together with the inner membrane of the autophagosome.

Compelling studies have helped to unravel the importance of autophagy in cell biology. Indeed, this catabolic pathway ensures in all cell-types the normal turnover of all organelles and most of long-lived proteins and it is up-regulated upon physiological (hormones) and environmental stress (nutrient starvation, hypoxia, infections...) conditions (*2, 3*).

It is well documented that toxic injury causes the formation of large autophagic vacuoles, associated with cell destruction when the damages are too important. Based on these features, this catabolic process has long been blamed as a second form of programmed cell death, in addition of the well-characterized type I apoptosis. Instead, recent evidence suggests that autophagy provides a protective signal that controls cell modeling throughout development, enable cell survival during starvation, and prevents cell aging (*4*) and transformation (*5*, *6*) during life. Consistently, defects of autophagy have been reported in breast, ovarian, and prostate cancers (*7*). Conversely, hyperactivation of autophagy has been involved in myopathies, and a number of neurodegenerative diseases such as Alzheimer's-, Parkinson's-, and Prion -diseases (*1*).

While the morphology and regulation of autophagy have been investigated extensively for animals, including humans, very little is known about the molecular machinery underlying this process (*8*). Emerging evidence in human intestinal cancer cells indicates that the formation of autophagosomes is stimulated by multiple signaling pathways as diverse as the GTPase Gᵢ₃ (*9*), the class III phosphatidylinositol 3-kinase (*9*), and the MAPK ERK (extracellular signal-regulated kinases 1 and 2) (*10*). However, as these signaling pathways are activated by myriad of stimuli, most of them are not linked to autophagy; further information is needed to understand how cell may fine-tune autophagy. This issue is important because regulating such underlying signaling might open therapeutic promises for treating diseases or conditions involving a deregulation of autophagy.

The present invention addresses this issue, and identifies that the MAPK kinases/CFTR pathway is a key component of the process of autophagy, and more particularly of the maturation stage of the autophagic process. New means enabling the regulation of the autophagy signaling pathway are thus provided by the present invention.
Furthermore, these new means have enabled the inventors to identify cystic fibrosis as an autophagic disease.

### CFTR and Cystic Fibrosis (CF):

CFTR is a member of the ABC (ATP-binding cassette) superfamily that transports chloride (Cl⁻) ions across membranes of epithelial cells of lungs, intestines, sweat duct, pancreas, liver, kidney, reproductive tract, and other fluid-transporting tissues.

As indicated by its acronym, the CFTR gene was identified as the genetic basis of the hereditary disease cystic fibrosis (*11*, *12*). The CFTR gene is found in region q31.2 on the long (q) arm of human chromosome 7. It spans 250,000 base pairs (bp) and is composed of 27 exons (13). The mRNA transcript for CFTR is 6129 bp long (see the NCBI sequence record NM_000492 to access the mRNA sequence data). 4443 bp within the mRNA (CDS sequence) code for amino acid sequence of CFTR.

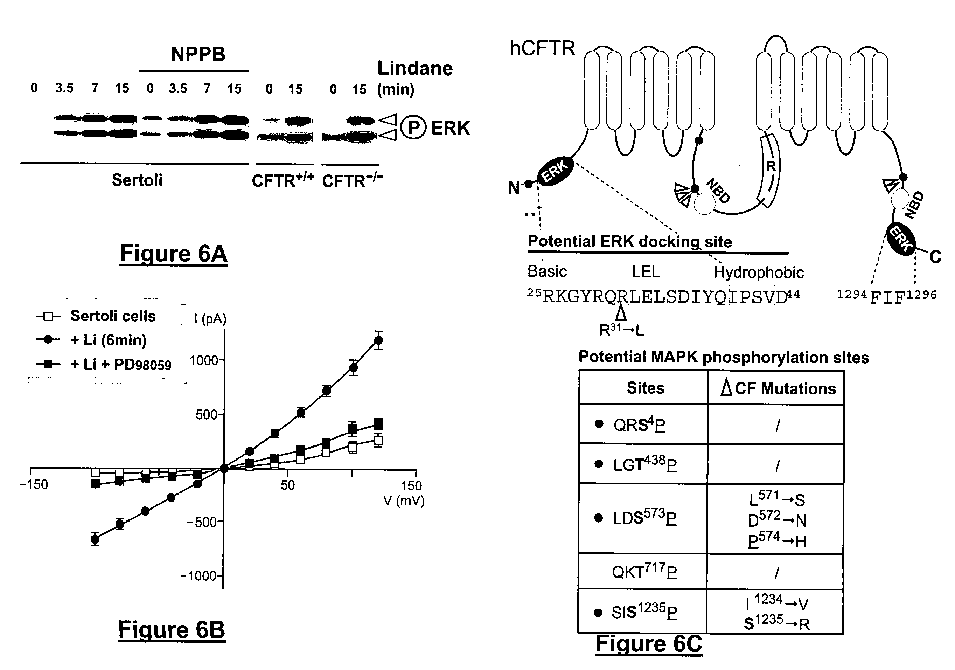

The CFTR protein is a glycoprotein of 1480 amino acids (see the CFTR protein sequence record in Swiss-Prot under accession number P13569; http://us.expasv.org/cgi-bin/niceprot.pl?P13569), containing two six-membrane-spanning domains, two nucleotide -binding domains (NBD), as well as a unique regulatory (R) domain (Fig. 6C).

So far, opening of CFTR channel has been shown to be controlled only by PKA-mediated phosphorylations of its R domain and ATP (adenosine triphosphate) binding and hydrolysis by the two NBD (reviewed in (14)).

Consistently, CFTR is a cAMP activated Cl⁻ channel found in apical membranes. It is also crucial for the regulation of other ion channels and transports, including the Epithelial Na⁺ Channel (ENaC), the Outwardly Rectifying Cl⁻Channel (ORCC), the basolateral K⁺ channels (ROMK1...), and the aquaporins *(15).* Besides its function as a channel, CFTR is a receptor for *Pseudomonas aeruginosa*, the major pathogen in CF airway *(16, 17).* It is also detected in hypothalamic neurons *(18, 19),* in the heart (20) and some lymphocytes (21) where its function is still undefined.

Defective versions of this protein, caused by CFTR gene mutations, are responsible for development of cystic fibrosis (CF), a frequent deadly disease (1 in 2500 birth) (22). Inherited as autosomal recessive disorder, CF is characterized by a generalized exocrinopathy of all organs that express *CFTR.* Important CF clinical features include malnutrition, chronic pulmonary disease, pancreatic deficiency, liver fibrosis, diabetes mellitus, cholelithiasis, male infertility (congenital bilateral aplasia of the vas deferens), and arthritis. The most striking pathological changes are seen in airways, in which inflammation, chronic infections and obstructive mucous secretion are responsible for lethal respiratory insufficiency.

Over 1000 different mutations in the CFTR gene have been reported to the CF Genetic Analysis Consortium database (www.genet.sickkids.on.ca/cftr/). About 70% of the mutations are found to be delta F508 (deletion of the amino acid phenylalanine located at position 508 within NBD1), making it the most common CF mutation. People who are homozygous for ΔF508 mutation tend to have the most severe CF symptoms.

To date, the exact physiological role of CFTR and the sequence of events leading to airway disease are not fully understood.
Detailed *biochemical* and *physiological* analyses of the WT and mutated CFTR have shown that the normal CFTR protein is synthesized in the endoplasmic reticulum (ER), transported to Golgi apparatus for additional processing before being integrated into the cell membrane. By contrast, once the ΔF508 CFTR mutant reaches the ER, the quality-control mechanism recognizes that the protein is folded incorrectly and marks the defective protein for rapid degradation. As a result, the affected cells completely lack CFTR and display altered secretion of electrolytes and fluid.

Based on these *in vitro* results, the prevailing model is that in normal epithelium, CFTR functions principally as a Cl⁻ channel *at the cell surface* where it drives by increasing the osmotic gradient, the secretion of water into the lumen. The hydrated low viscous mucus can then be effectively removed by cilia lining the lungs. In this "high salt" hypothesis, the loss of CFTR Cl⁻ secretion in CF membranes coupled with abnormal sodium absorption results in iso- or hyper-osmotic salt concentrations between the lumen and the epithelial cells. This impairs water flux, leading to the production of viscous mucus layer that fails to be removed by cilia and traps the bacteria. The mucosal dehydration, coupled with inflammatory and infective byproducts, creates thick and tenacious mucus that clogs and damages airways. In support of this hypothesis, a high Cl⁻ concentration of 60 mEq/1 or greater is measured in sweat of CF patients, which constitutes the CF diagnostic test [quantitative pilocarpine iontophoresis test]. Moreover, CFTR ensures in the colon the entire apical Cl⁻ conductance and hence the massive fluid loss in cAMP-mediated diarrhea such as cholera (23). Consistently, the notion that CFTR is absolutely required for fluid secretion came from the demonstrations that *newborn* CF patients and CFTR-deficient mice suffer of severe intestinal obstruction.

Nevertheless, this concept does not fit the puzzling observations that CF patients maintain normal Cl⁻ concentrations in their lungs *(24).* More intriguingly, at birth, the lung of the CF patients appears normal (*25, 26*) and develops pathological changes with a course and a severity that vary substantially amongst patients, even with similar CFTR genotypes.
**This implies that another mechanism must exist to explain how defective CFTR leads to airway disease and the involvement of environmental factors in CF progression.**

### SUMMARY OF THE INVENTION:

Starting from the demonstration that a widely-used pesticide Lindane (gamma-benzene hexachloride) is an inducer of autophagy, the inventors demonstrate that CFTR is a key component of the autophagy signaling pathway.
It is further demonstrated that activation of CFTR by phosphorylation induces and/or stimulates autophagy. It is more particularly demonstrated that CFTR phosphorylation induces and/or stimulates the maturation stage of autophagy, whereby autophagosomes fuse to the lysosomes to become autolysosomes.
The present invention thus provides new biological and medical applications for diseases involving a deregulation in autophagy.
As shown in the appended claims, the present invention more particularly provides new diagnosis and/or prognosis tools, as well as new preventive and/or palliative and/or therapeutic tools for these diseases.

The present invention also identifies for the first time cystic fibrosis as an autophagic disease.

### BRIEF DESCRIPTION OF THE FIGURES:

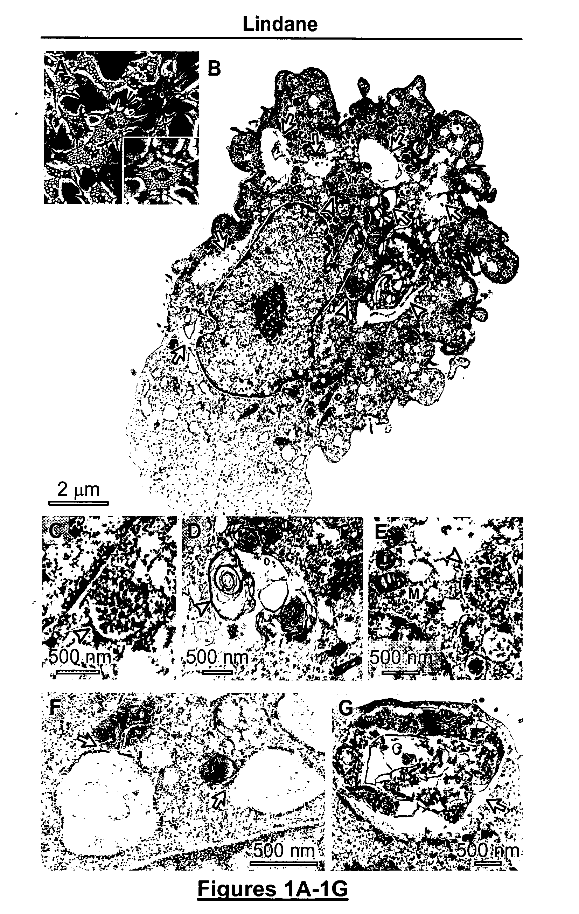

**Fig. 1.** Lindane induced accumulation of large autophagic vacuoles in Sertoli cells.
   **(A)** Cell treatment with Lindane (50 µM, 24 h) induced formation of vacuoles, readily observed under light microscope *(insert,* vacuolation started within 8 h of treatment in perinuclear region).
   **(B)** Representative electron microscopy photographs of Lindane-treated cells. Note the presence of numerous autophagic vacuoles from newly formed sequestrating membranes (arrowheads; C, high magnification), lamellar structures (arrowheads; **D,** high magnification), double-membrane autophagosomes (arrowheads; **E),** autophagosomes in close vicinity with late endosomes and lysosomes (arrows; **F),** to autolysosomes containing electron-dense degraded materials (arrows; **G,** high magnification). On average, ~ 50 autophagic vacuoles of 2-20 µm² were observed per Lindane-treated cells (n = 20), whereas little if any autophagosomes were found in untreated cells (n = 25).

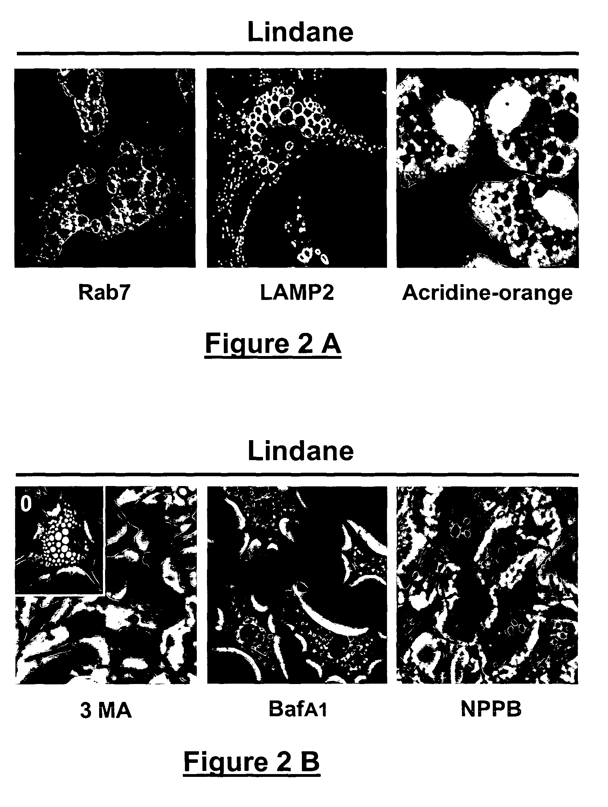

**Fig 2.** Lindane-induced autolysosomal vacuolation is dependent on V-ATPase pumping and Cl⁻ conductance.
   **(A)** Lindane-induced autophagic vacuoles were associated with markers of late endosomes (EGFP-Rab7 transfection) and lysosomes (anti-LAMP2 immunostaining), and were acidic (red fluorescence after acridine-orange staining). In contrast, morphology of early endosomes was not altered by Lindane.
   **(B)** Sertoli cells were pretreated 90 min with either vehicle (0), 3 MA (autophagy inhibitor, 30 mM), Baf_{A1} (V-ATPase inhibitor, 100 nM) or NPPB (Cl⁻ channel blocker, 25 µM) before Lindane addition. After 24 h, cells were observed under a light microscope and representative photographs are shown.

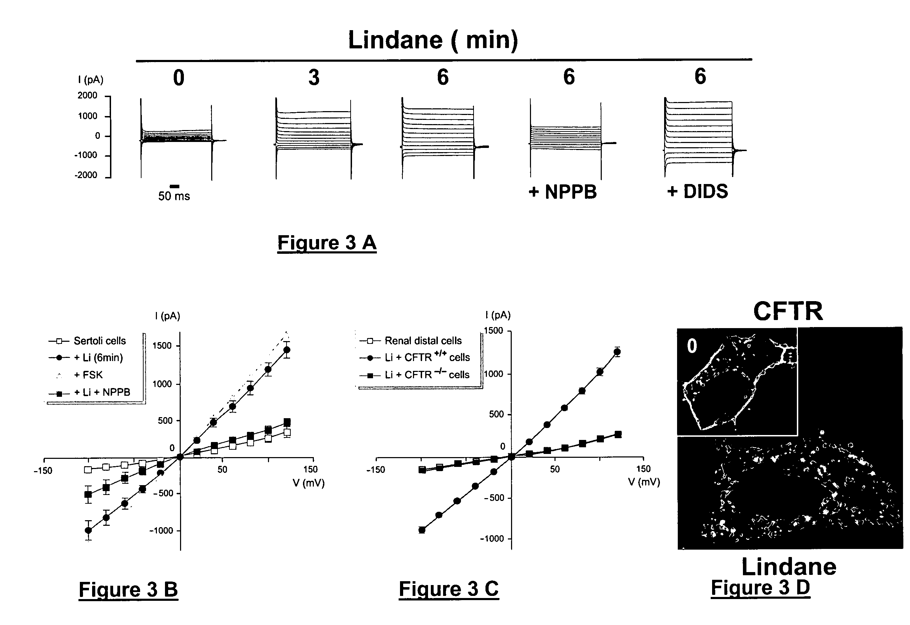

**Fig. 3.** CFTR Cl⁻ channel is mandatory for Lindane-induced autophagic vacuolation.
   **(A)** Electrophysiological properties of Lindane-stimulated Cl⁻ currents. Representative traces of whole cell currents from Sertoli cells before (0) and after stimulation with Lindane (Li, 50 µM) in the absence or the presence of NPPB (0.1 mM) or DIDS (1 mM).
   **(B)** Whole-cell current-voltage (*I*-*V*) relationship obtained from n = 8 Sertoli cells in each group before (□) and after stimulation with Lindane (○), Lindane plus NPPB (■), or forskolin (10 µM, ▲).
   **(C)** I-V curves in CFTR^{-/-} and CFTR^{+/+} renal distal cells *(n* = 8 cells in each group) before and after stimulation with Lindane. Note that reversed potentials for Lindane-induced currents were at ~0 mV (*n* = *8*), which is Cl⁻ equilibrium potential with the symmetrical solutions used. Results are means±S.D. of at least three experiments with n cells.
   **(D)** Confocal microscopy pictures of BHK cells showing partial redistribution of CFTR-EGFP from plasma membrane in basal conditions *(insert)* to vacuoles after Lindane treatment (24 h).
**Fig. 4.** Representative electron microscopy analyses of Lindane-treated CFTR^{-/-} cells showing accumulation of lamellar structures (arrowheads) with little if any large autophagic vacuoles (arrows) in contrast to Lindane-treated CFTR^{+/+} cells.
**Fig. 5.** Sustained ERK pathway activation is necessary and sufficient to commit cell to autophagic vacuolation.
   **(A)** Sertoli cells were stimulated with Lindane, lysed at indicated times and analyzed for ERK activation (arrowheads) by Western blotting. Densitometry scanning of Lindane-induced p42^{ERK2} phosphorylation is shown in the left.
   **(B)** Sertoli cells were pretreated with vehicle or PD98059 (MEK1 inhibitor, 10 µM, 90 min), before Lindane addition. 24 h later, vacuole formation was examined under light microscopy (*left upper photograph,* cells treated with PD98059 plus Lindane; *insert,* cells treated with Lindane alone) or electron microscopy. At the ultrastructural level, note in the presence of PD98059 and Lindane the small size of vacuoles (arrows) and the accumulation of lamellar structures (arrowheads) morphologically similar to those seen in Lindane-treated CFTR^{-/-} cells. High magnification *(left lower photograph)* showed that the contents of these membranous bodies were indistinguishable from the cytosol; highly suggesting that they were formed by the repeated sequestration of autophagosomes. As control, PD₉₈₀₅₉ did not affect chloroquine-induced vacuolation (500 µM, 24 h), ruling out unspecific side-effects on V-ATPase.
   (C) Transfection of MEK1⁺ mutant is sufficient to induce swelling of Rab7/LAMP2 vacuoles through downstream activation of Cl⁻ channels and V ATPase. Sertoli cells were transfected with MEK1⁺-HA plasmid and allowed to recover for 24 h before addition of vehicle, BafA1 (100 nM) and NPPB (25 µM) for 24 h. 48 h after transfection, morphology of rab7 (cotransfection of EGFP-Rab7 and MEK1⁺ plasmids) and of LAMP2 (anti-LAMP2 staining) compartments was analysed by confocal microscopy of HA-positive cells.
**Fig. 6.** CFTR is the downstream target of ERK that relays autophagic signal.
   **(A)** Lindane triggers ERK activation independently of CFTR. Sertoli, CFTR^{+/+} and
      CFTR^{-/-} renal cells were pretreated or not with NPPB (25 µM, 90 min) before Lindane addition. At the indicated times, cells were lysed and ERK activation was analyzed by Western blotting.
   **(B)** Inhibition of Lindane-induced CFTR Cl⁻ conductance by abrogation of ERK pathway. Whole-cell I-V relationships were obtained from control (□), PD98059-(identical to control I-V), Lindane- (○) or Lindane plus PD98059 (■) -incubated Sertoli cells (n = 8 cells in each group).
   (C) Potential ERK-docking and -phosphorylation sites within CFTR sequence.

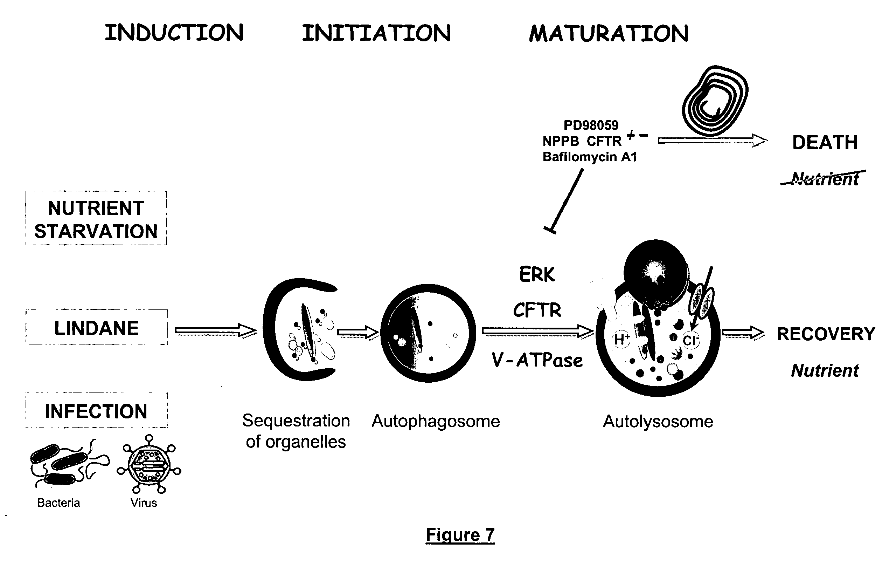

**Fig. 7.** A model for the control of autophagy by the ERK/CFTR/V-ATPase pathway.

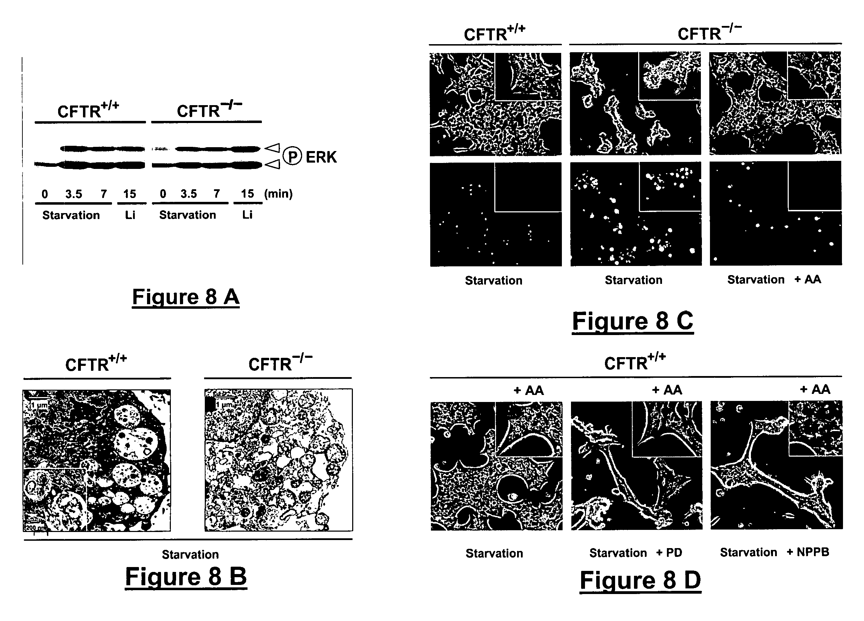

**Fig. 8.** Induction of autophagic cell death under nutrient deprivation by disruption of the CFTR/ERK pathway.
   **(A)** Rapid activation of ERK by starvation in CFTR^{+/+} and CFTR^{-/-} cells.
   **(B)** Ultrastructural features of starved CFTR^{-/-} cells. CFTR^{+/+} cells and CFTR^{-/-} cells were incubated in the absence of serum and nutrients for 6 h, and examined by electron microscopy. Note that the starved CFTR^{+/+} cells displayed extensive autophagic vacuolation with all profiles from early lamellar structures to autolysosomes *(insert).* By contrast, the starved-CFTR^{-/-} cells exhibited marked autophagic degenerative changes with accumulation of lamellar bodies, fragmented endoplasmic reticulum and swollen mitochondria with unclear christae, while the morphology of nucleus remained intact.
   **(C)** Starvation induces CFTR^{-/-} cell death which can be blocked by autophagy inhibitors. Compare the healthy starved CFTR^{+/+} cells with the dying starved CFTR^{-/-} cells which were round, loosely attached to the support *(insert,* high magnification) and labeled by propidium iodide (2 µg/condition, *lower panel*). A time course indicated that death began at 3-6 h after nutrient deprivation and was complete by 16-20 h. Addition of amino acids or 3 MA (data not shown) which inhibits autophagy initiation arrested starvation-induced cell death.
   **(D)** Representative pictures of CFTR^{+/+} cells treated for 16 hours under nutrient deprivation in the presence of PD98059 or NPPB. *Insert,* addition of amino acids blocked cell death in both conditions (PD + Starvation or NPPB + Starvation).

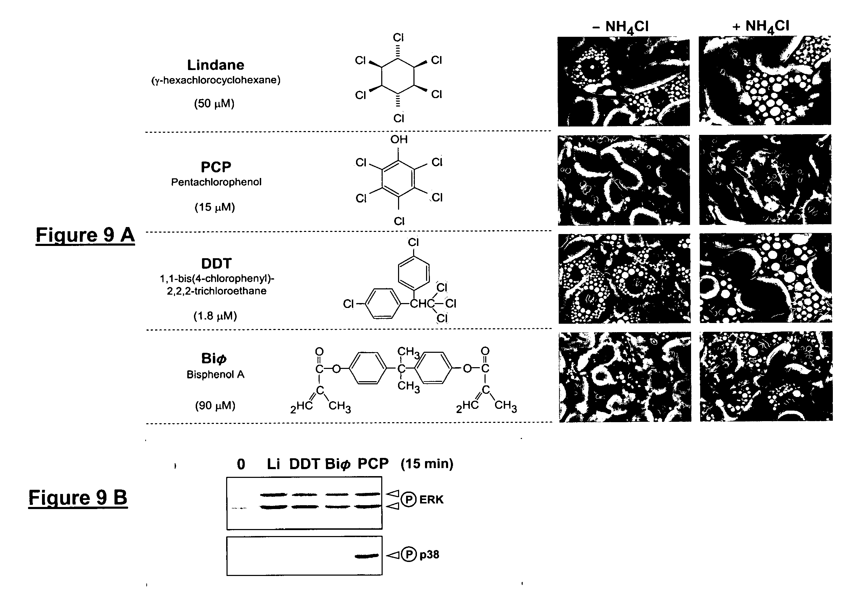

**Fig. 9.** Sertoli cell vacuolation is an early cellular response induced by several xenobiotics that is correlated to ERK activation.
   The responses of Sertoli cells to the indicated chemicals were examined on vacuole formation **(A,** cells treated for 24h) and on MAPK phosphorylations **(B,** Western blotting of cell lysates treated for 15 min). Note that the addition of NH₄Cl (5 mM) to medium which did not alter cell morphology, accelerated toxicant-induced vacuole development and allowed greater sensitivity in detecting vacuoles.

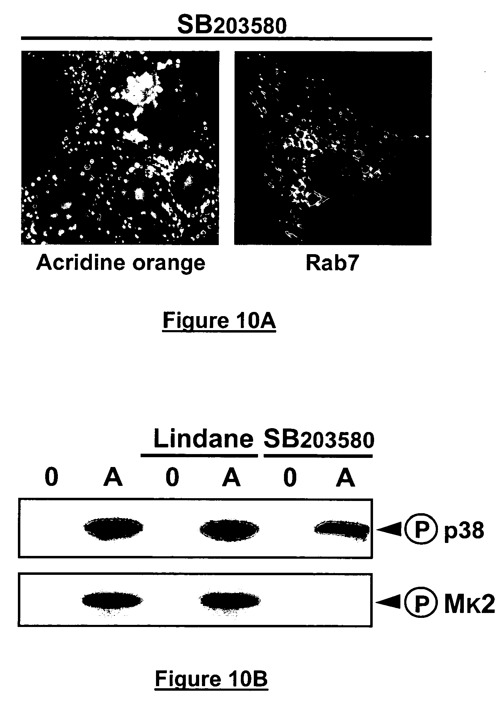

**Fig. 10.** Induction of Sertoli cell vacuolation by P38 inhibition.
   **(A)** SB₂₀₃₅₈₀ (p38-specific inhibitor, 10µM) as Lindane induced the accumulation of large acidic (acridine orange staining) Rab7⁺ vacuoles.
   **(B)** Lindane-induced autophagic vacuolation is not ascribed to p38 inhibition. Sertoli cells were pretreated with Lindane (50 µM, 5 h or 24 h) before being incubated for 15 min with the p38 activator Anisomycin (A, 10 µg/ml, 15 min). Western blotting analysis of the phosphorylation levels of p38 (anti-P p38, *upper panel*) and of its substrate, MAPKAP kinase 2 (P MK2, *lower panel*) were then performed as readout of p38 activation and p38 activity, respectively. The positions of the phosphorylated p38 and MK2 are indicated by arrowheads. As controls, SB₂₀₃₅₈₀ did impede Anisomycin-induced p38 activity.

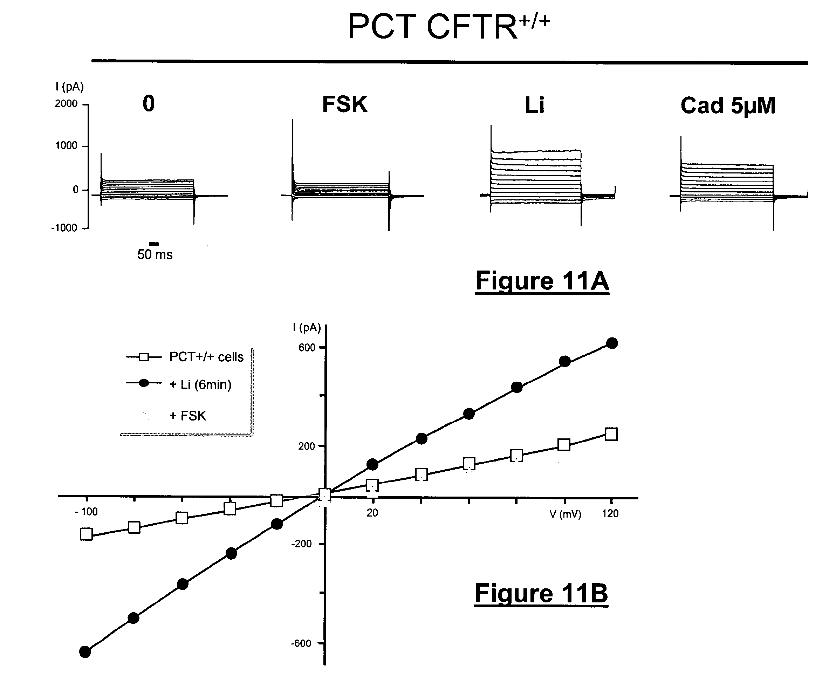

**Fig. 11.** Lindane activates CFTR Cl⁻ channel independently of PKA activation.
   **(A)** Electrophysiological properties of Lindane-stimulated Cl⁻ currents. Representative traces of whole cell currents from CFTR^{+/+} renal proximal PCT cells before (0) and after stimulation with Lindane (Li, 50 µM). Similar results were obtained with Cadmium a toxic heavy metal that does activate ERK.
   **(B)** Whole-cell current-voltage (*I-V*) relationship obtained from *n* = 8 renal proximal cells (PCT) in each group before (□) and after stimulation with Lindane (○), or forskolin (10 µM, ▲). Note that PKA activation by Forskolin failed to induce CFTR Cl⁻ currents. Results are means±S.D. of at least three experiments with n cells.

### DETAILED DESCRIPTION OF THE INVENTION:

The present invention demonstrates that CFTR (Cystic Fibrosis Transmembrane conductance Regulator) is a key component of the autophagy signaling pathway.

More particularly, CFTR is shown to be an essential actor of the autophagy maturation process: it is indeed demonstrated that CFTR expression or activity is essential for the maturation of autophagosomes into autolysosomes.

Delineating this pathway led to the demonstration that the mitogen-activated protein kinase (MAPK)/ERK pathway is involved in CFTR activation.
A chemical activator of this MAPK ERK/CFTR pathway is also provided, namely gamma-benzene hexachloride (also known as Lindane).

Most importantly, the loss of CFTR expression or activity, or the inhibition of the ERK pathway result in the accumulation of lamellar bodies (formed by the repeated sequestration of autophagosomes) and autophagic cell death under amino acid starvation.
The critical role played by CFTR in the control of autophagy, as well as its fine tuning by kinase pathways, open new diagnostic and therapeutic fields of applications.
It thus provides new means for the diagnosis and/or prognosis, as well as for the preventive and/or palliative and/or therapeutic treatment of diseases or conditions involving a deregulation in autophagy.
Such diseases or conditions notably comprise *cancer,* such as epithelial cancer, which involve *insufficient* autophagy initiation (7). Such diseases or conditions also comprise *degenerative diseases,* such as a neurodegenerative disease or a myopathy, which involve *excessive* autophagy initiation and *insufficient* autophagy maturation (1).

The present invention also provides new means to assess the autophagic stress that a compound or other external factor may induce, which is of the most importance for autophagy-sensitive organisms.
These means may notably help in the design of drugs showing reduced deleterious side effects.

The present invention further identifies for the first time cystic fibrosis as an autophagic disease. Cystic fibrosis is a lethal disease known to involve defects in the production or function of CFTR, a cAMP-regulated Cl⁻ channel in epithelial cells. It is the first time that a defect in autophagy is shown to be involved in cystic fibrosis. This autophagic model helps in understanding the development of the disease from the bad prognostic associated with amino acid deprivation due to gastrointestinal defects, to the accumulation of mucus and the lethal *respiratory insufficiency.*
The characteristic gastrointestinal defects shown by cystic fibrosis patients may hence be one of the causative agents responsible for accumulation of mucus in the lungs. It is very much likely that these gastrointestinal defects lead to important deprivations in amino acids, which in turns induce and stimulate autophagy. Moreover, during the course of the disease the chronic *bacterial infections* in the lungs of CF patients represent important inducers of autophagy that amplify the production of mucus (3, 22).
As cystic fibrosis is characterized by a mutated CFTR, some of the kinase/CFTR pathways are most probably unavailable for phosphorylation: according to the inventors' model of cystic fibrosis, it is very much likely that autophagy maturation is either blocked or insufficiently stimulated in cystic fibrosis patient.
The present invention thus has a new approach of cystic fibrosis, and identifies it as an autophagic disease, involving insufficient autophagy maturation and a comparatively excessive autophagy initiation.
This unbalance thereby leads to an increased sensitivity to autophagic death induced either by starvation or bacterial infection and to dramatic accumulation of lamellar bodies, forming the characteristic mucus observed in the lungs of cystic fibrosis patients.

The present inventors more generally give a common explanation to the development of those diseases or conditions that involve an excessive mucous production, such as allergy, infection, infertility, and suggest that these diseases involve a deregulation in CFTR activation resulting in an insufficient maturation and a comparatively excessive initiation of the autophagic process.

The present invention hence relates to a process for identifying a compound useful forthe manufacture of a drug intended for the preventive and/or palliative and/or therapeutic treatment of cystic fibrosis.
By the expression "compound useful for the manufacture of a drug", it is herein meant that said compound may be useful as a drug by its own, or in association with another active ingredient.

Such a process comprises screening for a compound that is capable of:
a) inhibiting the initiation of autophagy in a eukaryotic cell, and/or of
b) inducing and/or stimulating the maturation of autophagy in a eukaryotic cell,
where a positive determination at said step a) and/or b) identifies said compound as being useful in the manufacture of a drug intended for the preventive and/or palliative and/or therapeutic treatment of cystic fibrosis.
More particularly, a process for identifying a compound useful for the manufacture of a drug intended for the preventive and/or palliative and/or therapeutic treatment of cystic fibrosis may comprise providing a candidate compound, and
a) contacting said candidate compound with a growth factor-deprived and nutrient-deprived eukaryotic CFTR^{-/-} cell, and determining whether said candidate compound prevents or delays the autophagic death of said cell,
   and/or
b) contacting said candidate compound with a eukaryotic CFTR+/+ cell, and determining whether said candidate compound induces and/or stimulates at least one among the following features:
   b1) the formation of autophagic vacuoles in said eukaryotic CFTR +/+ cell,
   b2) the formation of CFTR-expressing vacuoles within the cytoplasmic compartment of said eukaryotic CFTR +/+ cell,
   b3) the delocalization of CFTR proteins from the plasma membrane to the cytoplasmic compartment of said eukaryotic CFTR +/+ cell,
      and/or
c) determining whether said candidate compound is capable of phosphorylating a CFTR protein, and/or of stimulating such a phosphorylation, and/or of inducing such a phosphorylation,
where a positive determination at said step a) and/or b) and/or c) identifies said compound as being useful for the manufacture of a drug intended for the preventive and/or palliative and/or therapeutic treatment of cystic fibrosis.

### Autophagic Vacuoles

Any means that the person of ordinary skill in the art find appropriate to determine whether autophagy is induced and/or stimulated may be used.
The formation of autophagic vacuoles is a feature of an undergoing autophagic process.

Said means hence notably include contacting said candidate compound with a eukaryotic cell, and detecting or monitoring the formation of autophagic vacuoles within the cytoplasm of this cell. The invention demonstrates that such cell needs to be CFTR+/+, if it is intended to monitor a normal autophagy maturation step.

Detection or monitoring autophagy is usually performed by **transmission electron microscopy**, by detecting the below typical autophagic structures (*27*).

Autophagic vacuoles comprise all vacuoles formed during the autophagic process from the initial stage vacuoles, known as autophagosomes, to the final stage vacuoles, known as autolysosomes.
*Autophagosomes* can be recognized as being double-membrane-enclosed vesicles, which contain undigested cytoplasmic material including organelles, and which are dramatically reduced by inhibitors of the initiation stage of autophagy such as 3 methyladenine (3-MA, available from Sigma, (*28*), amino acid, or RNAi to Atg (Autophagy) genes (Atg5, Atg9...(*29*)).
*Autolysosomes* are single-membrane vesicles which may still contain some cytoplasmic components at various stages of degradation. Their formation is inhibited by V-ATPase specific inhibitor, such as bafilomycin A1 (BafA1, available from Sigma (*30*)).
*Lamellar bodies* are formed by the repeated sequestration of autophagosomes.

Of course, there is a whole range of intermediary stages: between the initial autophagosome stage until the final autolysosome stage.

Alternatively or complementarily, **molecular markers** are available for such structures.
For example, LAMP2 is a marker of lysosomes and autolysosomes (anti LAMP2 antibodies are available from Pharmigen).
Several proteins have been described as being useful markers of autophagosomes.
For example, a mammalian protein termed LC3 has initially been identified as a protein that co-purifies with microtubule-associated protein 1A and 1B from rat brain (*71*). LC3 shows a 28% amino acid identity with a yeast protein termed ApgB/Aut7p, which is essential to the formation of autophagosomes (31). The amino acid sequence of rat Map1LC3 is available from Genbank™ under accession number U05784.
Two forms of rat LC3 have been described: LC3-I and LC3-II. It has been demonstrated that upon autophagy induction, the cytosolic LC3-I is processed to LC3-II which specifically associates with autophagosome membranes (32, 33). On SDS-PAGE, LC3-II (apparent mobility, 16 kD) migrates faster than LC3-I (apparent mobility, 18 kD). Consequently, measurement of LC3-II levels by immunoblotting is a method for determining the early autophagic activity of mammalian cells (32).

Three human orthologs of rat Map1LC3 have been identified and termed MAP1LC3A, MAP1LC3B, MAP1LC3C respectively; the three human isoforms are associated with autophagosome membranes (*34*) (the sequence of human Map1A/1B LC3 is available from Genbank™ under accession number NP_073729).
Furthermore, transgenic mice systematically expressing LC3 fused to a fluorescent detection marker have been generated and shown to be very useful for detecting and monitoring autophagy (35).
Hence, Apg8 and LC3 are very useful markers for autophagosomes in yeast and mammal autophagy, respectively.

Such markers can hence be used to detect these typical structures. Observation may then be performed by microscopy, e.g. confocal microscopy.
Transgenic mice expressing LC3 fused to a fluorescent marker, such as described in Mizushima *et al*. 2004 (*35*), may also be used as an *in vivo* non-human mammal model for monitoring autophagy.

The autophagic vacuoles in said above-mentioned step a1) hence notably comprise autophagosomes and/or autolysosomes. They preferably comprise autolysosomes.

### Autophagy activity: proteolysis of long-life proteins

In contrast to the proteasome which degrades short-lived proteins, autophagy accounts for the degradation of long-lived proteins. Therefore, measurement of degradation of long-lived proteins is often used to monitor autophagic activity.
To this end, cells are incubated with [¹⁴C]-valine for 18 h to label all cellular proteins.
After washing, cells are then chased with culture medium containing cold Val.
After 12 h incubation, at which time short-lived proteins are being degraded, the chase medium is replaced with fresh culture medium (with nutrient; negative control experiment) or nutrient-free Hanks' medium (starve+; positive control experiment) in the absence or the presence of said candidate compound. The incubation is continued for an additional 4-h period.
Then trichloroacetic acid (TCA)*-soluble radioactivity* in the culture supernatant, which corresponds to that of amino acids and peptides, is measured. In addition, the TCA soluble radioactivity within cells can be measured if necessary.
The accumulated radioactivity represents degradation of long-lived proteins.

### Delocalization of CFTR into the cytoplasm:

The present invention demonstrates that autophagic vacuoles express CFTR proteins and more particularly that the membrane of said autophagic vacuoles contains CFTR proteins. Induction and/or stimulation of autophagy hence results in an accumulation of, or an increase in CFTR proteins within the cytoplasmic compartment of said eukaryotic CFTR+/+ cell.

Procedures enabling to determine or monitor the subcellular localization of CFTR are known to the skilled person. It may e.g. comprise:
*Method 1*: Indirect immunofluorescence staining:
   - providing a CFTR-expressing cell seeded on a glass coverslip
   - contacting it with said candidate compound,
   - at the end of the incubation time needed to induce autophagy (2-24h), fixing the cells with methanol for 7 min at -20°C and permeabilizing them for 5 min at room temperature with saponin buffer (0.5 % saponin, 2.5% goat serum, 1% bovine serum albumin, and 0.2% gelatin in PBS),
   - using this saponin buffer throughout the experiment, incubating the fixed and permeabilized cells overnight with anti-CFTR antibodies at 4°C (5 µg/ml, R&D Systems (*36*), washing, and revealing with fluorescein isothiocyanate-conjugated anti-rabbit (Dako) antibodies for 1 h at room temperature.
   - analysing by confocal microscope (Leica) the staining of CFTR obtained in the presence of said Anti-CFTR, and the level obtained in the absence of said antibodies (negative control experiment). The cells incubated with a control immunoglobulin show no staining.
*Method 2:* Alternatively, epithelial cell line expressing CFTR fused to a fluorescent marker (*37*), such as described in example 1 Fig 3D, may also be used as a useful cellular model for monitoring the traffic of CFTR by confocal microscopy.

In both methods, determining whether the cytoplasmic level induced in the presence of said candidate compound is significantly superior to the level observed in the negative control experiment (obtained in the absence of said candidate compound).

### Phosphorylation of CFTR:

Procedures enabling to determine whether a candidate compound is capable of phosphorylating a CFTR protein, and/or of stimulating such a phosphorylation, and/or of inducing such a phosphorylation are known to the skilled person. They may e.g. comprise:
*Method 1: In vitro* kinase assay:
   - providing a soluble CFTR protein, a recombinant CFTR fragment or an immunoprecipitated CFTR proteins from lysates of CFTR-expressing cell,
   - contacting it with said candidate compound,
   - after a 15-min incubation at 25°C in the presence of [γ-³²P]ATP (Amersham; 4 µCi), stopping the reaction by the addition of reduced Laemmli buffer, heating the products at 100°C for 5 min and resolving the radiolabeled proteins by SDS-PAGE,
   - measuring by autoradiography the level of phosphorylated CFTR proteins obtained in the presence of said candidate compound, and the level obtained in the absence of said candidate compound (negative control experiment),
   - determining whether the level induced in the presence of said candidate compound is significantly superior to the level observed in the negative control experiment,
      Where a significantly superior level of CFTR phosphorylation identifies said candidate compound as capable of phosphorylating a CFTR protein, and/or of stimulating such a phosphorylation, and/or of inducing such a phosphorylation.
*Method 2*: *In vivo* CFTR phosphorylation:
   - labeling a CFTR-expressing cell with [³²P]orthophosphate (500 µCi/100mm petri) in phosphate-free DMEM for 5 hours,
   - contacting the labeled cells with said candidate compound for 15min, before being subjected to cell lysis with NP40/Bridj buffer as described (38) and supplemented with phosphatase and protease inhibitor cocktail,
   - immunoprecipitating the lysates with the anti CFTR or control antibodies,
   - resolving the [³²P]-phosphorylated proteins by SDS-PAGE, electroblotted onto PVDF membrane and visualized by autoradiography,
   - measuring by autoradiography the level of phosphorylated CFTR proteins obtained in the presence of said candidate compound, and the level obtained in the absence of said candidate compound (negative control experiment),
   - determining whether the level induced in the presence of said candidate compound is significantly superior to the level observed in the negative control experiment.
      Compounds which are capable of phosphorylating a CFTR protein, and/or of stimulating such a phosphorylation, and/or of inducing such a phosphorylation can be broadly referred to as CFTR activators.
      Such CFTR activators may act directly on CFTR by phosphorylating it, or may act indirectly on CFTR by activating a kinase which will in turn phosphorylate CFTR.
      The present invention demonstrates that CFTR can be activated through a MEK/ERK pathway.
      Hence, such CFTR activators include kinases such as ERK (e.g. activated ERK1, ERK2, and more particularly human ERK1, human ERK2), as well as kinase activators, such as ERK activators.

According to an aspect of the present invention, phosphorylation of CFTR is thus monitored with respect to its ERK phosphorylation sites. Said above-mentioned step c) may hence comprise determining whether said candidate compound is capable of phosphorylating a CFTR protein on at least one ERK phosphorylation site, and/or of stimulating such a phosphorylation, and/or of inducing such a phosphorylation.
When said CFTR protein is a human CFTR protein, said at least one ERK phosphorylation site can be selected from the group consisting of S4, T438, S573, T717, S1235.

### CFTR-/- eukaryotic cell:

Wild-type normal eukaryotic cell are CFTR+/+ cells. CFTR-/- cells can be derived therefrom by the skilled person following any routine procedure known in the art.
Eukaryotic CFTR-/- cells may e.g. be obtained as described in "Materials and Methods" of example 1.

### Growth factor-deprived and nutrient-deprived cells:

By "growth factor-deprived and nutrient-deprived cell" or "starved cell", it is herein meant a cell which has not been supplied in nutrient and growth factor for duration sufficiently long to induce autophagy.
Appropriate durations can be determined by the skilled person by trial and error experiments. They may slightly vary depending of the particular cell type being tested.
Eukaryotic CFTR+/+ cells usually require to be submitted to autophagy for at least 2 hours to initiate autophagy, but die after 24 hours of starvation. Usually a starvation of 10 to 15 hours is thus performed (e.g. overnight starvation).

Of course, if the cell being tested is a CFTR^{-/-} cell, autophagic cell death will occur in shorter times, e.g. in about 3-6 hours for a CFTR^{-/-} DCT cell.

Said eukaryotic CFTR^{+/+} or CFTR^{-/-} cell preferably is a CFTR^{+/+} or CFTR^{-/-} epithelial cell. More preferably, said cell is a CFTR^{+/+} or CFTR^{-/-} DCT cell (in that case, optional addition of NH₄Cl may facilitate observation of vacuoles). Most preferably, it is a CFTR^{+/+} or CFTR^{-/-} Sertoli cell (vacuoles are quite large within such cells; they are therefore easy to detect or monitor).

According to a preferred embodiment of the invention, the process for identifying a compound useful for the manufacture of a drug intended for the preventive and/or palliative and/or therapeutic treatment of cystic fibrosis further comprises determining whether said candidate compound is capable of inducing and/or stimulating a CFTR-regulated anionic and/or cationic current in a eukaryotic CFTR+/+ cell, such as notably a CFTR Cl⁻ conductance. Other ion conductance or transporters (such as the Na+ Channel ENaC, the outwardly rectifying Cl⁻ channel, the K⁺ channels ...) are also regulated by CFTR, and may thus represent an appropriate CFTR-regulated anionic and/or cationic current for said process.

As demonstrated by the present invention, illustrative said candidate compound for implementation of the process of the invention notably comprise activators of the MEK/ERK/CFTR pathway, and more particularly:
- activated kinases, such as activated ERK (activated ERK1 and/or ERK2, e.g. activated human ERK1 and/or ERK2), or activated PKA,
- kinase activators, such as activated MEK (e.g. activated MEK1), or gamma-benzene hexachloride, or any derivative thereof, which is obtainable by chemical substitution, but has retained the capacity of acting as an inducer or stimulator of autophagy maturation.
   The present invention indeed demonstrates that gamma-benzene hexachloride (also known as Lindane) acts as a kinase activator, and that it is capable of inducing autophagy in a eukaryotic cell. It is hence capable of inducing and/or stimulating the initiation of autophagy, as well as the maturation of autophagy.
   As demonstrated by the present invention, illustrative said candidate compound for implementation of the process of the invention also comprise kinase inhibitors (see example 2 below), such as p38 inhibitors (e.g. SB203580; available from Calbiochem).

According to a preferred embodiment of the present invention, the process for identifying a compound useful for the manufacture of a drug intended for the preventive and/or palliative and/or therapeutic treatment of cystic fibrosis further comprises selecting those candidate compounds which stimulate cell differentiation (e.g. compounds which induce sustained ERK activation (39) and/or discarding those candidate compounds which stimulate cell proliferation.

In line with the demonstration that CFTR is a key component of the autophagy signaling pathway, and notably of the maturation stage of autophagy, the present invention also provides a process for identifying an inducer or stimulator of autophagy maturation, as well as processes for identifying an inhibitor of autophagy initiation and/or a restorer of autophagy maturation.

According to an embodiment of the present invention, a process for identifying an inducer or stimulator of autophagy maturation, may comprise providing a candidate compound, contacting said candidate compound with a eukaryotic CFTR+/+ cell, and:
a) determining whether said candidate compound induces and/or stimulates the formation of CFTR-expressing vacuoles within the cytoplasmic compartment of said cell,
   and/or
b) determining whether said candidate compound induces and/or stimulates the delocalization of CFTR proteins from the plasma membrane to the cytoplamic compartment of said cell,
   where a positive determination at said step a) and/or b) identifies said compound as being an inducer or stimulator of autophagy maturation.

According to another embodiment of the present invention, a process for identifying an inducer or stimulator of autophagy maturation, may comprise:
a) providing a candidate compound,
b) determining whether said candidate compound is capable of phosphorylating a CFTR protein on at least one ERK phosphorylation site, and/or of stimulating such a phosphorylation, and/or of inducing such a phosphorylation,
   where a positive determination at said step b) identifies said compound as being an inducer or stimulator of autophagy maturation.
   Preferably, said CFTR protein is a human CFTR protein, and said at least one ERK phosphorylation site is selected from the group consisting of S4, T438, S573, T717, S1235.

According to another embodiment of the present invention, a process for identifying an inducer or stimulator of autophagy maturation, may comprise:
a) providing a candidate compound,
b) determining whether said candidate compound is capable of phosphorylating a CFTR protein, and/or of stimulating such a phosphorylation, and/or of inducing such a phosphorylation,
c) contacting said candidate compound with a eukaryotic CFTR+/+ cell, and determining whether said candidate compound induces and/or stimulates at least one among the following features:
   - the formation of autophagic vacuoles in said cell,
   - the formation of CFTR-expressing vacuoles within the cytoplasmic compartment of said cell,
   - the delocalization of CFTR proteins from the plasma membrane to the cytoplasmic compartment of said cell,
where a positive determination at both said steps b) and c) identifies said compound as being an inducer or stimulator of autophagy maturation.

Each of the three above-mentioned embodiments may further comprise determining whether said candidate compound induces and/or stimulates a CFTR-regulated anionic and/or cationic current, such as notably a CFTR Cl⁻ conductance in a eukaryotic CFTR^{+/+} cell. The information given in the above "autophagic vacuoles", "delocalization of CFTR into the cytoplasm", and "phosphorylation of CFTR", of course also applies to the present embodiments.

According to still another embodiment of the present invention, a process for identifying an inducer or stimulator of autophagy maturation, characterized in that it comprises:
a) providing a candidate compound,
b) determining whether said candidate compound is capable of phosphorylating a CFTR protein, and/or of stimulating such a phosphorylation, and/or of inducing such a phosphorylation,
c) determining whether said candidate compound induces and/or stimulates a CFTR-regulated anionic and/or cationic current in a eukaryotic CFTR+/+ cell, such as notably a Cl⁻ conductance. Other ion conductance or transporters (such as the Na⁺ Channel ENaC, the outwardly rectifying Cl⁻ channel, the K⁺ channels ...) are also regulated by CFTR, and may thus represent an appropriate CFTR-regulated anionic and/or cationic current for said process.
where a positive determination at both said steps b) and c) identifies said compound as being an inducer or stimulator of autophagy maturation.
In line with the demonstration that activated ERK acts as a CFTR activator, and thereby induces and/or stimulates autophagy maturation, said step a) may e.g. comprises determining whether said candidate compound is capable of phosphorylating a CFTR protein on at least one ERK phosphorylation site, and/or of stimulating such a phosphorylation, and/or of inducing such a phosphorylation. Said candidate compound may then be an activated ERK (e.g. activated human ERK1, activated human ERK2), or and ERK activator (e.g. activated MEK, or gamma-benzene hexachloride).

According to still another embodiment of the present invention, a process for identifying a compound which is an inducer or stimulator of autophagy maturation, may comprise:
a) providing a candidate compound,
b) contacting a CFTR+/+ eukaryotic cell with said candidate compound,
c) determining whether said candidate compound induces and/or stimulates a CFTR-regulated cationic and/or anionic conductance in said cell (compared to the level of conductance observed in the absence of said candidate compound, i.e. in the negative control),
   where a positive determination at said step c) identifies said candidate compound as an inducer or stimulator of autophagy maturation.
   Said CFTR-regulated cationic and/or anionic conductance may e.g. be a CFTR Cl-conductance.

### CFTR-regulated anionic and/or cationic current:

Any means that the skilled person finds appropriate to detect the induction and/or stimulation of said CFTR-regulated anionic and/or cationic current is appropriate. Such CFTR-regulated anionic and/or cationic currents notably comprise CFTR Cl⁻ conductance. Other ion conductance or transporters (such as the Na⁺ Channel ENaC, the outwardly rectifying Cl⁻ channel, the K⁺ channels ...) are also regulated by CFTR, and may thus represent an appropriate CFTR-regulated anionic and/or cationic current for said process.

Appropriate means to measure CFTR Cl⁻ conductance may e.g. comprise measuring the Cl⁻ currents or conductance across the plasma membrane of an eukaryotic cell, preferably a CFTR^{+/+} eukaryotic cell. Whole cell currents can be monitored with amplifier as previously described (*40*); see also example 1 below).

Induction and/or stimulation is assessed by comparison with the CFTR Cl⁻ control conductance that is measured under the same experimental conditions but in the absence of said compound.
To identify CFTR Cl⁻ conductance, the ruptured-patch whole-cell configuration of the patch-clamp technique is used. Patch pipettes (resistance 2-3 MΩ) are filled with a solution containing in (mM) 140 NMDGC1 (*N*-methyl-D-glucamine chloride), 1 or 5 EGTA, 5 MgATP, and 10 HEPES (N-2- hydroxyethylpiperazine-N'-2-ethanesulfonic acid) pH 7.4. The bath solution contains in (mM) 140 NMDGC1, 1 CaCl₂, 60 mannitol, and 10 HEPES, pH 7.4. These solutions are designed to study Cl⁻ currents because Cl⁻ is the sole permeant ion in these conditions and to prevent Ca²⁺ activated Cl⁻ currents by the addition of EGTA in the patch solution. After establishment of whole cell configuration, cells are stimulated by the addition of FSK (10 µM).

Figure 3A in example 1 shows typical recording from a cell expressing the CFTR Cl⁻ channel. The specificities of CFTR Cl⁻ conductance over other Cl⁻ conductances are the following (for review see (*41*):

### Biophysical Properties

1. Their activity is stimulated by cAMP (Forskolin) and by intracellular nucleotides.
2. They have a small single-channel conductance of 6-10 pS.
3. The relationship between the intensity of the current and the voltage applied to the cell (I-V) is linear, when bathed in symmetrical Cl⁻ concentrations.

### Ion Selectivity

3. They are selective for anions over cations.
4. The anion permeability sequence is Br⁻≥ Cl⁻> I. This anion permeability sequence distinguishes CFTR Cl⁻ channels from other epithelial Cl⁻ channels that have a higher permeability to I⁻ than Cl⁻, for example, the outwardly rectifying Cl⁻ channel. They are blocked when Cl⁻ is replaced by I⁻ in the bath solution, with a shift of the E rev (reversion potential) towards the more positive values.

### Pharmacology of Block

5. They are sensitive to NPPB (5-nitro-2-(3-phenylpropylamino) benzoic acid, 0.1 mM), glibenclamide (0.1 mM) but not to external DIDS (4-4'Diisothiocyanostilben-2-2'disulfonique acid, 1 mM). The failure of disulfonic stilbenes (such as DIDS) to inhibit CFTR Cl⁻ channels when added to the extracellular side of the membrane distinguishes CFTR from other epithelial Cl⁻ channels that are blocked by extracellular DIDS.

ENAC: According to the classical cell model of Na⁺ transport in tight epithelia, Na⁺ enter the cell across the apical membrane by diffusion through a Na⁺-selective apical conductance. These epithelial Na⁺ channels (ENaC) is characterized at the channel level in the apical membrane, using the patch clamp technique described above. ENaC is defined i) by a high ion cationic selectivity (permeability ratio *P*_{Na}/*P*_{K}>20), *ii*) a reversed potential at ~+58 mV : the reversal potential of Na+, *iii)* a low unitary conductance (5 pS in the presence of Na⁺), *iv*) gating kinetics characterized by long closing and opening times and v) a high sensitivity to amiloride (*K*ᵢ: 0.1 µM).

K⁺ is the most abundant cation in the intracellular compartment and is required for many normal functions of the cell. K+ currents are measured using the whole cell patch clamp mode described above. Typically, CFTR regulates several K+ transporters and channels (e.g. ROMK). ROMK channels exhibit biophysical properties comparable to those of the low-conductance K+ channel i) a single-channel conductance of 30-40 pS in symmetrical KCl solutions, *ii*) high K+ selectivity (current is blocked by replacing intracellular K⁺ with Cs⁺, potential reversion at -100mV), *iii*) high channel open probability at physiological potentials, *iii*) weak inward rectification, *iv*) marked sensitivity to external Ba²⁺ but not to TEA, marked sensitivity to intracellular pH, inhibition by arachidonic acid, v) channel rundown or loss of channel activity in excised patches in the absence of MgATP that involves dephosphorylation by a protein phosphatase, and *vi*) inhibition by glibenclamide (i.e., when ROMK2 is coexpressed with CFTR).

According to still another embodiment of the present invention, a process is provided for identifying an inhibitor of autophagy initiation and/or a restorer of autophagy maturation. This process may comprise:
a) providing a candidate compound,
b) contacting an eukaryotic CFTR-/- cell with said candidate compound,
c) inducing the initiation of an autophagic process in said CFTR-/- cell,
d) determining whether said candidate compound inhibits or delays the autophagic death of said eukaryotic CFTR-/- cell, (compared to the level observed in the absence of said candidate compound, i.e. in the negative control)
   where a positive determination at said step d) identifies said candidate compound as an inhibitor of autophagy initiation and/or as a restorer of autophagy maturation.

The present invention identifies that the process of autophagy comprises an initiation step and a maturation step, and that each of these stages can be up- or down-regulated. The present invention further identifies that an excessive autophagy initiation coupled to a defective autophagy maturation leads to an excessive production of autophagic structures.

By "autophagic structures", it is herein intended those characteristic autophagic structures which are known by the skilled person under the name of autophagic vacuoles (and notably autophagosomes), autophagic lamellar bodies, secretion granules of surfactant. The present invention hence encompasses a process for identifying a compound useful for the manufacture of a drug intended for the preventive and/or palliative and/or therapeutic treatment of a disease or condition involving an excessive **production of autophagic structures**, which comprises:
- identifying a compound which induces and/or stimulates autophagy maturation by a process according to the invention, or
- identifying a compound which restores autophagy maturation and/or inhibits autophagy initiation by a process according to the invention,
where such an identified compound is a compound useful for the manufacture of a drug intended for the preventive and/or palliative and/or therapeutic treatment of a disease or condition involving an excessive production of autophagic structures.
The present invention demonstrates that a cell that cannot properly achieve autophagy maturation accumulates lamellar autophagic structures (e.g. secretion granules of surfactant). A compound or a factor inducing autophagy maturation or inhibiting autophagy initiation in such a cell will prevent the accumulation of such structures.

The present invention further identifies that diseases or conditions involving an excessive production of autophagic structures notably comprises those diseases or conditions which involve an excessive mucus production.

According to an advantageous embodiment of the invention, a disease or condition which involves an excessive mucus production is cystic fibrosis.
Said disease or condition involving an excessive production of autophagic structures and more particularly an excessive production of mucus may alternatively be:
- asthma, rhinosinusitis, allergy, or an infertility disease or condition, or
- a disease or condition involving at least one viral and/or bacterial and/or fungal infection.

The present invention further identifies that diseases or conditions involving an excessive production of autophagic structures also comprises those diseases or conditions which involve the undesired death of a non glandular eukaryotic cell, such as e.g. a neuronal cell or a muscular cell. In such a case, the death of the non glandular eukaryotic cell is caused by an excessive autophagy initiation coupled to a proportionally insufficient autophagy maturation. Illustrative diseases or conditions involving an excessive production of autophagic structures in a non glandular eukaryotic cell notably comprise degenerative disease, more particularly a neurodegenerative disease or condition (e.g. Alzheimer's disease, Parkinson's disease, Huntington's disease, Creutzfeldt-Jakob's disease), or a myopathy (e.g. cardiomyopathy).

The present invention further encompasses any use of an inducer or stimulator or restorer of autophagy maturation for the manufacture of a drug intended for the preventive and/or palliative and/or curative treatment of cystic fibrosis. Said inducer or stimulator of autophagy maturation may notably be a CFTR activator. Said CFTR activator may e.g. be capable:
- of phosphorylating a CFTR protein on at least one ERK phosphorylation site, and/or of stimulating such a phosphorylation, and/or
- of inducing such a phosphorylation, and/or of inducing the stimulation of such a phosphorylation.
   Such a CFTR activator can thus be an activated ERK (such as activated ERK1 and/or ERK2, e.g. activated human ERK1 and/or ERK2), or an ERK activator (such as an activated MEK, or gamma-benzene hexachloride (Lindane)).
   Preferably, said inducer or stimulator of autophagy maturation is capable of inducing and/or stimulating a CFTR-regulated cationic and/or anionic current in a eukaryotic CFTR+/+ cell, such as a CFTR Cl⁻ conductance...

Advantageously, said inducer or stimulator of autophagy maturation can be gamma-benzene hexachloride, or any derivative thereof, which is obtainable by chemical substitution, but has retained said capacity of acting as an inducer or stimulator of autophagy maturation. In that case, an antioxidant may advantageously further be used for the manufacture of said drug, said antioxidant and said gamma-benzene hexachloride or gamma-benzene hexachloride derivative being intended for simultaneous, separate or sequential administration.

The present also encompasses any **use of an inhibitor of autophagy initiation for the manufacture of a drug or dietary supplement** intended for the preventive and/or palliative and/or curative treatment of cystic fibrosis.
As the present invention identifies cystic fibrosis as an autophagic disease, cystic fibrosis patients may be deficient in one or several amino acids. Such deficiencies can be assessed by collection of a blood sample, analysis of its amino acid contents, and comparison to the normal standard levels observed in healthy patients.
According to an advantageous aspect of the present invention, said inhibitor of autophagy initiation hence comprises at least one aminoacid, or is an amino acid, or is a plurality of amino acids.
In the context of the present invention, 'amino acid residue' means any amino acid residue known to those skilled in the art (see e.g.: Sewald *et al*., 2002 (*42*); IUPAC nomenclature under http://www.chem.qmul.ac.uk/iupac/AminoAcid/).
This encompasses naturally occurring amino acids (including for instance, using the three-letter code, Ala, bAla, Arg, Asn, Asp, Cys, Gln, Glu, Gly, His, Ile, Leu, Lys, Met, Phe, Pro, Ser, Thr, Trp, Tyr, Val), as well as rare and/or synthetic amino acids and derivatives thereof (including for instance Aad, Abu, Acp, Ahe, Aib, Apm, Dbu, Des, Dpm, Hyl, MeLys, MeVal, Nva, HAO, NCap, Abu, Aib, MeXaa and the like (see e.g.: (Müller *et al*., 1993; Aurora *et al*., 1998; Obrecht *et al*., 1999; Maison *et al*., 2001; Formaggio *et al*., 2003;Nowick *et al*., 2003; (43-48).
Said amino acid residue or derivative thereof can be any isomer thereof, especially any chiral isomer, e.g. the L- or D- isoform.
By amino acid derivative, we hereby mean any amino acid derivative as known in the art (see e.g.: *Sewald et al*., 2002 (*42*); IUPAC nomenclature under http://www.chem.qmul.ac. uk/iupac/AminoAcid/).

For instance, amino acid derivatives include residues derivable from natural amino acids bearing additional side chains, e.g. alkyl side chains, and/or heteroatom substitutions. Further examples of amino acid derivatives comprise amino acid bearing chemical modifications such the one fund in mimetic peptides or peptidomimetics, which are compounds containing non-peptidic structural elements that are capable of mimicking or antagonizing the biological action(s) of a natural parent peptide. A peptidomimetic usually does no longer have classical peptide characteristics such as enzymatically scissille peptidic bonds.

Preferably, said aminoacid belongs to the group of the non-essential aminoacids. Preferred non-essential aminoacids are glycine, alanine, proline, serine, cysteine, tyrosine, asparagines, glutamine, aspartic acid, glutamic acid, arginine, histidine.

Appropriate aminoacids may be accurately selected by selecting those aminoacids which are in lower amounts in the patient into which the drug is to be administered. Dosage and administration regimen can be determined as a function of the patient's level in said aminoacid. Preferred dosage and administration regimen are those which intend to increase the patient's aminoacid level up the normal standard level.

Said drug or dietary supplement may further comprise a compound which inhibits the initiation stage of autophagy, such as 3-MA or any RNAi to Autophagy genes...

The present invention also relates to the use of gamma-benzene hexachloride, or of a derivative thereof which is obtainable by chemical substitution, but has retained said capacity of acting as an inducer or stimulator of autophagy maturation or as a CFTR activator, for the manufacture of a drug intended for the preventive and/or palliative and/or curative treatment of a disease or condition involving an excessive production of autophagic structures.
This use may further comprise using an antioxidant for the manufacture of said drug, wherein said gamma-benzene hexachloride or gamma-benzene hexachloride derivative, and said antioxidant are intended for simultaneous, separate or sequential administration.
Said disease or condition involving an excessive mucus production may e.g. be:
- cystic fibrosis,
- asthma, rhinosinusitis, allergy,
- a disease or condition involving at least bacterial and/or viral and/or fungal infection,
- an infertility disease or condition,
- a degenerative disease or condition.

Still in line with the demonstration that CFTR is a key component of the autophagy signaling pathway, and notably of the maturation stage of autophagy, the present invention also provides a process for identifying an inhibitor of autophagy maturation, as well as a process for identifying an inducer or stimulator of autophagy initiation.

Appropriate candidate compounds for the identification of an inhibitor of autophagy maturation notably comprise a CFTR protein, or a CFTR protein fragment, in the form of a soluble molecule.

By soluble molecule, it is intended a molecule that is not expressed in a cell, e.g. an isolated or synthetically produced cell-free molecule.
Preferably said CFTR protein fragment comprises a sequence which would correspond to an ERK phosphorylation site in a CFTR protein, such as a human CFTR protein.
Appropriate said CFTR protein fragment may also comprise a sequence which would correspond to an ERK docking site in a CFTR protein, such as a human CFTR protein.

According to an embodiment of the present invention, a process for identifying an inhibitor of autophagy maturation may comprise:
a) providing a candidate compound,
b) inducing autophagy in a eukaryotic CFTR+/+ cell,
c) contacting said candidate compound with said cell,
d) determining whether said candidate compound inhibits:
   - the formation of CFTR-expressing vacuoles within the cytoplasmic compartment of said cell, and/or
   - the delocalization of CFTR proteins from the plasma membrane to the cytoplamic compartment of said cell,
   where a positive determination at said step d) identifies said compound as being an inhibitor of autophagy maturation.

According to another embodiment of the present invention, a process for identifying an inhibitor of autophagy maturation may comprise:
a) providing a candidate compound,
b) determining whether said candidate compound is capable of inhibiting the phosphorylation of a CFTR protein on at least one ERK phosphorylation site, and/or of stimulating such an inhibition, and/or of inducing such an inhibition,
   where a positive determination at said step b) identifies said compound as an inhibitor of autophagy maturation.
   If said CFTR protein is a human CFTR protein, said at least one ERK phosphorylation site may be selected from the group consisting of S4, T438, S573, T717, S1235.
   The CFTR protein that is used as target in said step b) may be provided in non-isolated form, e.g. by providing a CFTR-expressing eukaryotic cell, whereas said candidate compound may be a CFTR protein, or a CFTR protein fragment, in the form of a soluble molecule.
   Of course, as the determination to be made is about knowing whether said candidate compound is capable of inhibiting the phosphorylation of a CFTR protein on at least one ERK phosphorylation site, and/or of stimulating such an inhibition, and/or of inducing such an inhibition, the target CFTR protein may be used as a full-length CFTR protein, as well as simply as a CFTR fragment which has retained at least one ERK phosphorylation site.
   Said target CFTR protein or ERK site-containing fragment can conveniently be expressed by a cell.
   An appropriate candidate compound would then be a CFTR soluble protein, or a CFTR soluble fragment which has retained at least one sequence of an ERK phosphorylation site.

According to another embodiment of the present invention, a process for identifying an inhibitor of autophagy maturation may comprise:
a) providing a candidate compound,
b) determining whether said candidate compound is capable of inhibiting the phosphorylation of a CFTR protein, and/or of stimulating such an inhibition, and/or of inducing such an inhibition,
c) inducing the formation of autophagic vacuoles in a eukaryotic CFTR+/+ cell, contacting said candidate compound with said cell, and determining whether said candidate compound inhibits:
   - the formation of autophagic vacuoles in said cell, and/or
   - the formation of CFTR-expressing vacuoles within the cytoplasmic compartment of said cell, and/or
   - the delocalization of CFTR proteins from the plasma membrane to the cytoplamic compartment of said cell,
   where a positive determination at both said steps b) and c) identifies said compound as being an inhibitor of autophagy maturation.

Each of the three above-mentioned embodiments may further comprise determining whether said candidate compound inhibits a CFTR-regulated cationic and/or anionic conductance, such as a CFTR Cl⁻ conductance in a eukaryotic CFTR+/+ cell.

According to still another embodiment of the present invention, a process for identifying an inhibitor of autophagy maturation may comprise:
a) providing a candidate compound,
b) determining whether said candidate compound is capable of inhibiting the phosphorylation of a CFTR protein, and/or of stimulating such an inhibition, and/or of inducing such an inhibition,
c) determining whether said candidate compound inhibits a CFTR-regulated cationic and/or anionic current (such as a CFTR Cl⁻ conductance...) in a eukaryotic CFTR+/+ cell,
where a positive determination at both said steps b) and c) identifies said compound as being an inhibitor of autophagy maturation.

Said step d) may advantageously comprise determining whether said candidate compound is capable of inhibiting the phosphorylation of a CFTR protein on at least one ERK phosphorylation site, and/or of stimulating such an inhibition, and/or of inducing such an inhibition. Said candidate compound may then advantageously be an ERK inhibitor or an inhibitor of the MEK/ERK/CFTR pathway.
The CFTR protein that is used as a target at said step b) may be provided in non-isolated form, i.e. expressed by a eukaryotic cell, whereas said candidate compound may be a CFTR protein, or a CFTR protein fragment, in the form of a soluble molecule.

According to still another embodiment of the present invention, a process for identifying an inhibitor of autophagy maturation, characterized in that it comprises:
a) providing a CFTR protein or a CFTR protein fragment,
b) determining whether said CFTR protein or CFTR fragment is capable of competing with an ERK for binding to a CFTR protein,
   where a positive determination at said step b) identifies said CFTR protein or CFTR fragment as an inhibitor of autophagy maturation.

According to still another embodiment of the present invention, a process for identifying a compound which is an inhibitor of autophagy maturation may comprise:
a) providing a candidate compound,
b) contacting a CFTR+/+ eukaryotic cell with said candidate compound,
c) determining whether said candidate compound inhibits a CFTR-regulated cationic and/or anionic conductance in said cell (compared to the conductance level observed in the absence of said candidate compound, i.e. in the negative control),
   where a positive determination at said step c) identifies said candidate compound as an inhibitor of autophagy maturation.
   Said CFTR-regulated cationic and/or anionic conductance may e.g. be a CFTR Cl⁻ conductance. Other ion conductance or transporters (such as the Na⁺ Channel ENaC, the outwardly rectifying Cl⁻ channel, the K⁺ channels ...) which are regulated by CFTR may represent an appropriate CFTR-regulated anionic and/or cationic current for said process.

According to still another embodiment of the present invention, a process for identifying a compound which is an inhibitor of autophagy maturation may comprise:
a) providing a candidate compound,
b) inducing autophagy in a eukaryotic CFTR+/+ cell,
c) contacting said candidate compound with said cell,
d) determining whether said candidate compound induces and/or stimulates the autophagic death of said cell,
   where a positive determination at said step d) identifies said compound as an inhibitor of autophagy maturation. As demonstrated by the present invention, illustrative said candidate compound for implementation of the process of the invention notably comprise inhibitors of V-ATPase (Bafilomycin,) of ERK (PD98059) and of CFTR channels (NPPB).

According to still another embodiment of the present invention, a process for identifying a compound which is an inducer or stimulator of autophagy initiation may comprise:
a) providing a candidate compound,
b) contacting said candidate compound with a eukaryotic CFTR-/- cell, and determining whether said candidate compound induces and/or stimulates the autophagic death of said cell,
where a positive determination at said step b) identifies said compound as an inducer or stimulator of autophagy maturation and/or an inducer or stimulator of autophagy initiation.

The processes of the invention for identifying an autophagy activator or stimulator, as well as the processes of the invention for identifying an autophagy inhibitor may further comprise isolating or purifying the identified autophagy activator or stimulator, or autophagy inhibitor, respectively.

The present invention further encompasses any *in vitro* use of gamma-benzene hexachloride, or of any derivative thereof obtainable by chemical substitution but has retained said capacity of acting as an inducer and/or stimulator of autophagy maturation or as a CFTR activator, to induce and/or stimulate autophagy in a eukaryotic cell.
In accordance with the present invention, gamma-benzene hexachloride and the conservative derivative thereof can thus be used to induce and/or stimulate the formation of autophagic vacuoles within the cytoplasmic compartment of a eukaryotic CFTR+/+ cell, or to induce and/or stimulate a CFTR-regulated cationic and/or anionic conductance, or to induce and/or stimulate the autophagic death of a eukaryotic CFTR-/- cell.

The present invention also encompasses a process for identifying a compound useful for the manufacture of a drug intended for the preventive and/or palliative and/or therapeutic treatment of a disease or condition which involves the undesired survival of a eukaryotic cell that shows an insufficient autophagy initiation, which comprises:
- identifying a compound which induces or stimulates or restores autophagy initiation by a process according to a process of the invention, and/or
- identifying a compound which inhibits autophagy maturation, thereby leading to autophagic death of the cell by a process according to a process of the invention,
where such identified compound is a compound useful for the manufacture of a drug intended for the preventive and/or palliative and/or therapeutic treatment of a disease or condition involving the undesired survival of a eukaryotic cell that shows an insufficient autophagy initiation.
Said disease or condition which involves an insufficient autophagy may e.g. be a cancer or tumor condition, such as an epithelial cancer or tumor.

The present invention also encompasses a process for identifying a compound useful for the manufacture of a drug intended for the preventive and/or palliative and/or therapeutic treatment of a disease or condition which involves the undesired death of a eukaryotic cell that shows an excessive autophagy initiation and an insufficient autophagy maturation, which comprises:
- identifying a compound which inhibits autophagy initiation and/or a restores autophagy maturation by a process according to the invention,
- identifying a compound which induces or stimulates autophagy maturation by a process according to the invention,
where such an identified compound is a compound useful for the manufacture of a drug intended for the preventive and/or palliative and/or therapeutic treatment of a disease or condition involving the undesired death of a eukaryotic cell that shows an excessive autophagy initiation and an insufficient autophagy maturation.
According to the present invention, said disease or condition which involves the undesired death of a eukaryotic cell that shows an excessive autophagy initiation and a defective autophagy maturation may e.g. be a degenerative disease, more particularly a neurodegenerative disease or condition (e.g. Alzheimer's disease, Parkinson's disease, Huntington's disease, Creutzfeldt-Jakob's disease), or a myopathy (e.g. cardiomyopathy).

To check whether a peptide corresponding to a CFTR fragment capable of acting as an inhibitor of autophagy maturation by inhibition or prevention of CFTR phosphorylation (through competition with ERK for binding or phosphorylating CFTR), a process for identifying an inhibitor of autophagy maturation according to the invention can be implemented with said fragment as a candidate compound.
The present invention hence encompasses any CFTR fragment which can be identified as an inhibitor of autophagy maturation by implementation of such a process of the invention.

More particularly, the invention relates to any peptide capable of acting as an inhibitor of autophagy maturation by inhibition or prevention of CFTR phosphorylation (through competition with ERK for binding or phosphorylating CFTR), characterized in that said peptide has a sequence that is identical to the sequence of a CFTR protein fragment, provided that said fragment has retained at least one of the ERK binding or phosphorylation sites contained in said CFTR protein.
Said CFTR protein preferably is a mammal CFTR protein, most preferably a human CFTR protein. ERK binding and phosphorylation sites of human wild-type CFTR protein are shown on Figure 6C.

According to an advantageous embodiment of the present invention, a peptide capable of acting as an inhibitor of autophagy maturation by inhibition or prevention of CFTR phosphorylation is characterized in that its sequence consists in a sequence selected from:

| | | |
|---|---|---|
| **SEQ ID NO:1** | **RKGYRQRLELSDIYQIPSVD** | **(ERK binding site)** |
| **SEQ ID NO:2** | **KVFIFSG** | **(ERK binding site)** |
| **SEQ ID NO:3** | **QKVFIFSG** | |
| **SEQ ID NO:4** | **QKVFIFSGT** | |
| **SEQ ID NO:5** | **PQKVFIFSGT** | |
| **SEQ ID NO:6** | **PQKVFIFSGTF** | |
| **SEQ ID NO:7** | **MQRSPLE** | **(ERK phosphorylation site)** |
| **SEQ ID NO:8** | **MQRSPLEK** | |
| **SEQ ID NO:9** | **MQRSPLEKA** | |
| **SEQ ID NO:10** | **MQRSPLEKAS** | |
| **SEQ ID NO:11** | **MQRSPLEKASV** | |
| **SEQ ID NO:12** | **SLLGTPV** | **(ERK phosphorylation site)** |
| **SEQ ID NO:13** | **SLLGTPVL** | |
| **SEQ ID NO:14** | **FSLLGTPVL** | |
| **SEQ ID NO:15** | **FSLLGTPVLK** | |
| **SEQ ID NO:16** | **FSLLGTPVLKD** | |
| **SEQ ID NO:17** | **YLLDSPF** | **(ERK phosphorylation site)** |
| **SEQ ID NO:18** | **YLLDSPFG** | |
| **SEQ ID NO:19** | **LYLLDSPFG** | |
| **SEQ ID NO:20** | **LYLLDSPFGY** | |
| **SEQ ID NO:21** | **LYLLDSPFGYL** | |
| SEQ ID NO:22 | YLSDSPF | mutated form |
| SEQ ID NO:23 | YLSDSPFG | |
| SEQ ID NO:24 | LYLSDSPFG | |
| SEQ ID NO:25 | LYLSDSPFGY | |
| SEQ ID NO:26 | LYLSDSPFGYL | |
| SEQ ID NO:27 | YLLNSPF | mutated form |
| SEQ ID NO:28 | YLLNSPFG | |
| SEQ ID NO:29 | LYLLNSPFG | |
| SEQ ID NO:30 | LYLLNSPFGY | |
| SEQ ID NO:31 | LYLLNSPFGYL | |
| SEQ ID NO:32 | YLLDSHF | mutated form |
| SEQ ID NO:33 | YLLDSHFG | |
| SEQ ID NO:34 | LYLLDSHFG | |
| SEQ ID NO:35 | LYLLDSHFGY | |
| SEQ ID NO:36 | LYLLDSHFGYL | |
| **SEQ ID NO:37** | **IVQKTPL** | **(ERK phosphorylation site)** |
| **SEQ ID NO:38** | **IVQKTPLQ** | |
| **SEQ ID NO:39** | **SIVQKTPLQ** | |
| **SEQ ID NO:40** | **SIVQKTPLQM** | |
| **SEQ ID NO:41** | **SIVQKTPLQMN** | |
| **SEQ ID NO:42** | **SFSISPG** | **(ERK phosphorylation site)** |
| **SEQ ID NO:43** | **SFSISPGQ** | |
| **SEQ ID NO:44** | **ISFSISPGQ** | |
| **SEQ ID NO:45** | **ISFSISPGQR** | |
| **SEQ ID NO:46** | **ISFSISPGQRV** | |
| SEQ ID NO:47 | SFSVSPG | mutated form |
| SEQ ID NO:48 | SFSVSPGQ | |
| SEQ ID NO:49 | ISFSVSPGQ | |
| SEQ ID NO:50 | ISFSVSPGQR | |
| SEQ ID NO:51 | ISFSVSPGQRV | |
| SEQ ID NO:52 | SFSIRPG | mutated form |
| SEQ ID NO:53 | SFSIRPGQ | |
| SEQ ID NO:54 | ISFSIRPGQ | |
| SEQ ID NO:55 | ISFSIRPGQR | |
| SEQ ID NO:56 | ISFSIRPGQRV, | |

or a conservative variant sequence thereof, which is obtainable by substitution or deletion, of at least one amino acid, but has retained a capacity of acting as an inhibitor of autophagy maturation.

The present invention also relates to any compound comprising such a peptide or peptide variant, provided that said compound is not a full length CFTR protein.

The present invention also relates to any nucleic acid coding for a peptide of the invention, taking into account the degeneracy of the genetic code.
Are also encompassed by the present invention any vector comprising such a nucleic acid, as well as any cell (such as a Sertoli cell or a DCT cell) transformed by such a vector.
The present invention also relates to any antibody which binds to CFTR on an epitopic site which comprises a sequence selected from SEQ ID NO: 1-21, 37-46 (normal CFTR).
The present invention also relates to any antibody which binds to CFTR on an epitopic site which comprises a sequence selected from SEQ ID NO: 22-36, 47-56 (mutated CFTR).
Such antibodies are useful for the detection of CFTR phosphorylation sites for ERK. They may also be used to prevent CFTR phosphorylation at these sites, and may then be used as CFTR inhibitors, i.e. autophagy inhibitors.

The present invention further encompasses any use of a CFTR inhibitor for the manufacture of an anti-tumor drug intended to induce autophagic cell death of chemo- or radio-resistant cells. A CFTR inhibitor will notably act as an inhibitor of autophagy maturation.
Said CFTR inhibitor may be an ERK inhibitor.
Advantageously, said CFTR inhibitor is a compound capable of inhibiting or preventing CFTR phosphorylation (e.g. through competition with ERK for binding to or for phosphorylating CFTR), and more particularly a peptide of the invention.

Said drug may further comprise a chemotherapeutic agent and/or radiotherapeutic agent for simultaneous, separate or sequential administration.
The present invention also relates to a pharmaceutical composition intended for the palliative and/or curative treatment of a resistance to anti-tumor chemotherapy or radiotherapy, characterized in that it comprises such a CFTR inhibitor. Such a pharmaceutical composition may further comprise a chemotherapeutic agent for simultaneous, separate or sequential administration.

The present invention also encompasses any **use of a CFTR activator** for the manufacture of a drug intended for the preventive and/or palliative and/or curative treatment of a disease or condition involving an excessive production of autophagic **structures (e.g. autophagosomes, lamellar bodies...) by a eukaryotic cell.**
Said disease or condition involving an excessive production of autophagic structures by a eukaryotic cell may be a degenerative disease or condition involving an excessive autophagy initiation and insufficient autophagy maturation, e.g. Alzheimer's disease, Parkinson's disease, Huntington's disease...
Advantageously, said CFTR activator can be gamma-benzene hexachloride, or any derivative thereof, which is obtainable by chemical substitution, but has retained said capacity of acting as an inducer or stimulator of autophagy maturation by activating CFTR phosphorylation. In that case, an antioxidant may advantageously further be used for the manufacture of said drug, said antioxidant and said gamma-benzene hexachloride or gamma-benzene hexachloride derivative being intended for simultaneous, separate or sequential administration.

The present invention also relates to the diagnosis and/or prognosis applications which derive from the demonstration that CFTR is a key component of the autophagy signaling pathway.
The presence hence relates to an ***in vitro* method for the diagnosis and/or prognosis of a disease or condition involving an excessive mucous production,** characterized in that it comprises detecting a deficient or defective autophagy, more particularly a defective autophagy maturation in a biological sample.
Said disease or condition involving an excessive mucous production may be cystic fibrosis.

Said disease or condition involving an excessive mucous production may be a pneumonia, bronchiolitis, asthma, rhinosinusitis, allergy, a disease or condition involving a bacterial and/or viral and/or fungal infection, an infertility disease or condition.

The present invention also encompasses an *in vitro* method for detecting an autophagy dysfunction in a eukaryotic CFTR-expressing cell, more particularly a dysfunction at the maturation stage of the process of autophagy. It notably comprises detecting whether there is a defective phosphorylation of a CFTR protein in said eukaryotic cell.
By defective phosphorylation, it is herein meant either an absence of phosphorylation, or a phosphorylation level that is significantly inferior to the level observed in a negative control experiment (i.e. the control phosphorylation level which would be observed in comparable but healthy eukaryotic cell).
Said eukaryotic cell advantageously is an epithelial cell.
According to an embodiment of the present invention, the in vitro method for detecting an autophagy dysfunction comprises detecting whether there is a defective phosphorylation of a CFTR protein on at least one of its ERK phosphorylation site.
According to an advantageous embodiment of the present invention, said CFTR protein is a human CFTR, and in that said at least one ERK phosphorylation site is selected from the group consisting of sites S4, T438, S573, T717, S1235 of human CFTR.

The present invention also encompasses *in vitro* methods for detecting an autophagy dysfunction in a eukaryotic CFTR-expressing cell (e.g. a blood cell; a lung cell; a renal cell; a Sertoli cell; ...).
According to an embodiment of the present invention, the *in vitro* method for detecting an autophagy dysfunction in a eukaryotic CFTR-expressing cell comprises detecting whether there is a defective binding of ERK to CFTR in said eukaryotic cell.
According to another embodiment of the present invention, the *in vitro* method for detecting an autophagy dysfunction in a eukaryotic CFTR-expressing cell comprises detecting whether there is a mutation of said CFTR in at least one of its ERK phosphorylation sites, and/or a mutation in at least one site involved in ERK binding to CFTR. If said CFTR is a human CFTR, the method may comprise detecting whether there is a mutation of said human CFTR in at least one site selected from:
- any site from R25 to D44 (see Figure 6C),
- sites Q2, R3, S4, P5,
- sites L436, G437, T438, P439,
- sites L571, D572, S573, P574,
- sites Q715, K716, T717, P718,
- sites S1233, I1234, S1235, P1236,
- sites F1294, I1295, F 1296.

The methods of the invention for detecting a dysfunction of autophagy can be applied to the diagnosis and/or prognosis of any disease or condition which involves an autophagy dysfunction: e.g. insufficient autophagy initiation such as an epithelial cancer or a defect in autophagy maturation such as a degenerative disease or cystic fibrosis.

The present invention more particularly relates to an *in vitro* method for the diagnosis and/or prognosis of a disease or a condition involving a mutated CFTR (e.g. a loss of function mutated CFTR), which comprises detecting in a biological sample whether there is an insufficient content in at least one amino acid, and/or an abnormal amino acid distribution.
According to the present invention, diseases or conditions involving a mutated CFTR notably comprises chronic pancreatic disease (e.g. pancreatitis), gastrointestinal disease, cystic fibrosis.
By the expression "abnormal distribution", it is herein meant a distribution that is significantly different from the control distribution which would be observed in a healthy subject. According to the present invention, cystic fibrosis patients are thought to usually have a sub-normal content in some or all amino acids.

The present invention further relates to an *in vitro* method to evaluate the level of autophagic stress that a compound or other external factor may induce in an autophagy-sensitive subject, characterized in that it comprises:
a) providing a CFTR deficient cell, such as a eukaryotic CFTR deficient cell,
b) *in vitro* exposing said CFTR deficient cell to said compound or factor, and
c) determining the level of autophagic cell death induced by said exposure (i.e. compared to a negative control),
where the level of autophagic cell death thus determined is in positive correlation with the said level of autophagic stress.
By CFTR deficient cell, it is herein intended a cell that either does not express CFTR (e.g. CFTR-/- cells) or a cell that does not express a sufficient level of CFTR or that expresses a defective CFTR (e.g. mutated CFTR). Said CFTR deficient cell may e.g. a CFTR deficient epithelial cell, for example a Sertoli CFTR-/- or a DCT CFTR-/- cell.
The present invention demonstrates that a CFTR-/- cell cannot achieve autophagy maturation. A compound or a factor inducing and/or stimulating autophagy initiation in such a cell will induce an accumulation of damaged organelles and debris, thereby leading to autophagic death of the cell.
The level of autophagic death of a CFTR deficient cell is hence representative of the level of autophagic stress to which said cell is submitted.

Said CFTR deficient cell may be provided in a sample collected from an animal suffering from a deregulation in autophagy signaling. For example, said CFTR deficient cell may be provided in a sample collected from an animal suffering from an excessive production of mucous, e.g. a non-human animal or a human being affected by cystic fibrosis, pneumonia, or allergy, or by an infertility condition. Said CFTR deficient cell may also be provided in a sample collected from a non-human animal or a human being suffering from cancer, or a degenerative disease...

The present invention further relates to a eukaryotic CFTR-/- cell, such as an epithelial CFTR-/- cell, preferably a DCT CFTR-/- cell, more preferably a Sertoli CFTR-/- cell.
It more particularly relates to a **kit to assess a risk of autophagic stress for autophagy-sensitive persons,** characterized in that it comprises at least one Sertoli CFTR-/- cell and/or DCT CFTR-/- cell.
Preferably, said eukaryotic CFTR-/- cell is an immortalized cell, e.g. an immortalized Sertoli or DCT CFTR-/- cell (cf. example 1 below).
The present invention also encompasses any non-human animal, such as e.g. a mouse, a rat, a rabbit, a pig or guinea-pig, which has been genetically engineered to produce or contain CFTR-/- cells.

The present invention further relates to the **use of a CFTR+/+ Sertoli or DCT cell for the detection and/or observation of an autophagic process.**

CFTR+/+ Sertoli or DCT cells indeed enable to easily detect and autophagic vacuoles. They therefore are useful tools for detection and/or monitoring of a reaction involving an autophagic process.
CFTR+/+ Sertoli cells are particularly advantageous, because the autophagic vacuoles they produce have a large size, and are thus easy to detect and/or monitor.
When CFTR+/+ DCT cells are used, NH₄Cl may be used to increase the size of the vacuoles, thus rendering detection and observation easier.
The present invention also encompasses a kit intended for the detection and/or observation of autophagy, which comprises at least one CFTR+/+ Sertoli cell or DCT cell, and NH₄Cl.

) Still in line with the demonstration that CFTR is a key component of the autophagy signalling pathway, the present invention provides an *in vitro* method to evaluate the level of deleterious side effects induced by a compound designed for therapy of cystic fibrosis. This method may comprise evaluating the level of autophagic cell death induced by *in vitro* exposure of a CFTR-/- cell to said compound.

According to a further aspect of the present invention, the present application also encompasses an *in vitro* process for assessing whether a drug comprising a p38 inhibitor may induce deleterious side effects when administered in an animal body, which comprises:
a) providing a eukaryotic cell,
b) *in vitro* contacting said cell with said p38 inhibitor-containing drug,
c) determining whether said drug induces and/or stimulates autophagy in said cell,
where a positive determination at said step c) indicates that said drug may induce deleterious side effects when administered in an animal body.
Said step c) may further comprise assessing the level of autophagy thereby induced or stimulated, this level being in positive correlation with the level of deleterious side effects induced by said drug.
Said drug may advantageously be a drug intended for the preventive and/or palliative and/or therapeutic treatment of inflammatory diseases, inflammation, arthritis, such as rheumatoid arthritis.

### EXAMPLE 1: Control of Autophagy by the ERK/CFTR Pathway

### Materials and Methods:

### Plasmids.

The expression plasmids for the following cDNA constructs have been described previously: EGFP-Rab7 (*49*), CFTR-EGFP (*37*), MEK1⁺ (a HA tagged dominant positive mutant of MEK1 in which the phosphorylation sites S²¹⁸ and S²²² are replaced with D) (50), and pSV3 neo (ATCC).

### Mice.

WT or CFTR^{-/-} mice were obtained from *Centre de Développement des Techniques Avancées pour l'Expérimentation Animale* (CNRS CDTA, Orléans, France). Animals were handled in accordance with guidelines of French Agricultural Office and legislation governing animal studies.

### Cell lines.

**BHK-21** (Baby hamster kidney epithelial cells, ATCC) stably expressing CFTR-EGFP cell-line was maintained in Dulbecco's modified Eagle's medium (DMEM, GIBCO) containing 10% fetal calf serum (GIBCO).

### Establishment of Sertoli 42GPA9 cell line

The Sertoli 42GPA9 cell line has been established from sexually mature polyoma virus large T (PyLT) transgenic mice.

### PyLT mouse strains (51)

pPyLT1 DNA (*52*) was microinjected into one fertilized egg from crosses between two C57BL/6 x DCA/2 (B6D2) F₁ parents according to standard techniques. DNA was injected either as complete circular molecules or after excision of the SalI-PvuI (fragments that contains most of the vector sequences), both procedures resulting in the same frequency of transgenic births, as well as in the same phenotypes. A total of 44 transgenic mice were obtained and families were maintained by serial crosses with normal B6D2 partners. Homozygous animals were generated in each family and always exhibited the same phenotype as the hemizygotes. In all of them, the transgene was expressed in the seminiferous epithelium of testis (Sertoli and germ cells), with no pathological consequences during most of the animals' lives. However, every old male developed large bilateral tumours of the testes, generated by the proliferation of Sertoli cell derivatives. Sertoli Cell lines could be readily established from the tumours and from the still apparently normal testis before the onset of tumoral growth.

### Establishment of 42GPA9 Sertoli cell line (53)

Three 6 month-old PyLT transgenic males (identified by tail dot blot analysis with a PyLT probe) were sacrificed by cervical dislocation and testes were excised aseptically. Then, Sertoli cells were prepared by sequential enzymatic digestion (15 min with Collagenase, DNAse I, Hyaluronidase, Trypsin, Sigma) as previously described (*54*).

Testes decapsulation Cut into small pieces Seminiferous tubules 0.5 mg/ml Collagenase, 5ml,
33°C, 15 min, continuous agitation or manual agitation every 5 min sedimentation for 5 min Leydig cells
(supernatant) and Seminiferous cords (Pellet) Wash 2x 0.5 mg/ml Trypsin + 1 μ/ml DNase,
5ml, 33°C, 15 min, continuous agitation pipetting gently with a Pasteur pipette to disperse
Seminiferous cells ↺ 80 x g for 10 min Wash Pellet 2x with PBS filter cells through a nylon (77 and
55 μm pores) Diluted in PBS/0.5% BSA. Sertoli + Germ cells Culture DMEM pyr, 6 mM lactacte 3 days to
obtain confluent monolayers in Matrigel dishes hypotonic solution (10mM tris HCl) to lyse germ cells
Sertoli cells

The Sertoli cells were plated at a density of 10⁶/well into 100-mm dishes (Falcon) and cultured in DMEM supplemented with 10% FCS, 100 U/ml penicillin, 100 µg/ml streptomycin at 32°C (GIBCO). The contaminating germ cells were removed by hypotonic lysis on day 3 of culture (54). Primary cultures of Sertoli cells were prepared according to the same protocol except that the cells were cultured in DMEM containing insulin (10 µg/ml), transferrin (5µg/ml). After 1 month, Sertoli cells from PyLT mice entered crisis. Many cells showed signs of necrosis and detached from the plate, while other cells began proliferating and formed numerous small colonies. Surviving cell colonies were removed from the plate with Trypsin (0.05%)-EDTA (0.53 mM) with the aid of cloning cylinders and were replated separately in 24-well plates. Cells were split at 1:4 dilutions. The immortalized cell lines obtained were subsequently cloned by limiting conditions. Briefly, cells were removed from the plate, diluted in DMEM containing 10% FCS until a single cell was present in each well of a 96-well culture dish. Colonies visible by phase-contrast microscopy were isolated and cells were grown to form monolayers. 42GPA9 cells were maintained in DMEM supplemented with 10% FCS at 32°C.

### Establishment of CFTR-/-cell lines

To address the importance of CFTR in autophagy, renal cells derived from distal tubules or the proximal convoluted tubules of Wild type (WT or CFTR^{+/+}) or CFTR deficient (CFTR^{-/-}) mice (40) were isolated and immortalized with the pSV3 neo vector, as described below (*55*).

### Primary Cell Cultures

3-4-week-old mice were killed by cervical dislocation, and kidneys were removed. Distal collecting tubules (DCT) and Proximal Convoluted Tubules (PCT) were microdissected under sterile conditions from kidneys. Kidneys were perfused with Hank's solution (GIBCO BRL) containing 700 U/ml Collagenase (Collagenase type2, Worthington Biochemical Corporation, # 4174), and were then cut into small pyramids that were incubated for 1 h at room temperature in the perfusion buffer (160U/ml Collagenase, 1% foetal calf serum―Dutscher, #P971410―, and 1 mM CaCl₂), and continuously aerated. Pyramids were then rinsed thoroughly in the same buffer devoid of Collagenase. Individual nephrons were dissected by hand in this buffer under binoculars, using stainless steel needles mounted on Pasteur pipettes.
After rinsing in the dissecting medium, tubules were transferred to collagen-coated 35-mm Petri dishes (rat tail collagen prepared in the laboratory according to the method of (*56*) filled with culture medium composed of DMEM/Ham's F12 (1:1, Sigma # D-0547) supplemented with 15 mM NaHCO₃, 20 mM HEPES, pH 7.4, 1% foetal calf serum (Dustcher, # P971410), 2 mM glutamine (In Vitrogen, # 25030-024), 5 µg/ml insulin (Sigma, # I-6634), 50 nM dexamethasone (Sigma, # D-4902), 10 ng/ml epidermal growth factor (Promega, # G-5021), 5 µg/ml Transferrin (Sigma, # T-2252), 30 nM sodium selenite (Sigma, # S-5261), and 10 nM triiodothyronine (Sigma, # T-6397). Cultures were maintained at 37°C in a 5% CO₂/95% air water-saturated atmosphere. The medium was removed 4 d after seeding and then every 2 days.

### Transformation of Primary Cultures With pSV3 neo

Fifteen-day-old primary cultures were transfected with pSV3 *neo* (ATCC) using the calcium phosphate technique. Twenty-four hours prior to transfection, monolayers were treated with trypsin and harvested cells were replated at a density of 1-2 x 10⁵ cells/cm². The calcium phosphate-DNA coprecipitate was prepared as follows: 19 µg of pSV3 *neo* plasmid in 10 µl Tris-EDTA buffer were dissolved in 490 µl of a 250 mM CaCl₂ solution. This solution was added drop-wise with gentle mixing to 500 µl of a solution containing 280 mM NaCl, 1.5 mM Na₂HPO₄, and 50 mM HEPES, adjusted at pH 7.08 with NaOH. A 160-µl aliquot of this solution was added drop-wise to each Petri dish containing 1.6 ml of culture medium (see above). Cells were incubated overnight in this solution at 37°C, rinsed, and incubated in culture medium. After 24 h, selection of the clones was performed by the addition of 500 µg/ml geneticin (In Vitrogen, G418 # 11811-031). Culture medium containing G418 was changed every day. Isolated geneticin resistant clones were picked, expanded, and assayed for CFTR expression by patch clamp (FSK-activated Cl⁻ currents) and RT-PCR experiments.
The renal epithelial cell lines that express or not CFTR are referred as CFTR^{+/+} and CFTR^{-/-} cells, respectively. They were maintained in DMEM/Ham's F12 (1:1, Sigma # D-0547) supplemented with 15 mM NaHCO₃, 20 mM HEPES, pH 7.4, 1% foetal calf serum (Dustcher, # P971410), 2 mM glutamine (In Vitrogen, # 25030-024), 5 µg/ml insulin (Sigma, # I-6634), 50 nM dexamethasone (Sigma, # D-4902), 10 ng/ml epidermal growth factor (Promega, # G-5021), 5 µg/ml transferrin (Sigma, # T-2252), 30 nM sodium selenite (Sigma, # S-5261), and 10 nM triiodothyronine (Sigma, # T-6397).

### Cell treatments.

Autophagy was induced either by starvation or by treatment with Lindane under nutrient-rich condition. For Lindane treatment, cells were serum-starved for 2-16 hours in DMEM/Ham's F12 (1:1, GIBCO #61965-026: F12 GIBCO #31765-027) supplemented with either 1% Insulin-Transferrin-SeleniumA (GIBCO #51300-044) or 0.1% bovine serum albumin (A7030, Sigma) and treated with Lindane (50 µM, Sigma, H4500). For CFTR-EGFP transfected BHK cells, NH₄Cl (5 mM) which did not alter cell morphology, was added to medium to allow detection of Lindane-induced vacuolation.

For nutrient deprivation, cells were washed with DMEM and first starved in minimal medium lacking serum for 90 min. Cells were then washed again with PBS and deprived for up to 16 h in nutrient-free medium lacking amino acids, vitamins, and serum (HBSS, 0.1% BSA, 20 mM HEPES, Sigma #H8264). As controls, cells were incubated in this nutrient-free medium supplemented with a mixture of non essential amino acids (0.1 mM, GIBCO, #11140-035).

When bafilomycin A1 (BafA1, V-ATPase-specific inhibitor, 100 nM, Sigma); Park Davis Inhibitor PD98059 (MEK₁-specific inhibitor, 10 µM, Cell Signaling), H89 (PKA inhibitor, 5 µM, Calbiochem), 3-Methyladenine (3 MA, autophagy-specific inhibitor, 10-30 mM, Sigma) or NPPB [5-nitro-2-(3-phenylpropylamino) benzoic acid, Cl⁻ channel blocker, 25 µM, Sigma] was used, it was added to the starvation medium for 90 min, prior to Lindane addition or nutrient deprivation. As controls, cells were incubated with vehicle (Me₂S0 or ethanol; 1:5000), anisomycin (p38 and JNK activator, 10 µg/ml, 15 min, Sigma), forskolin (FSK, PKA activator, 20 µM, 15 min, Sigma) or chloroquine (membrane-permeant weak base known to induce endosomal vacuolation, 500 µM, 24 h, Sigma).

### Electron microscopy.

The ultrastructural changes caused by Lindane or starvation in Sertoli and renal cells were analysed by transmission electron microscopy, as previously described (*57*). Briefly, control and Lindane-treated cells (50 µM, 24 h) were scraped from the culture dish in PBS EDTA 1mM, pelleted and fixed with ice-cold 2.5% glutaraldehyde (TAAB, #G004) in 0.1 M Na cacodylate, pH 7.4 for at least 1 h at 4°C. Cell pellets were then rinsed in cacodylate buffer, postfixed in 1% OsO₄ for 1 h at 4°C, dehydrated through graded ethanol washes, and embedded in epoxy resin. Oriented 1-mm sections were obtained with diamond knives, and representative areas were chosen for ultra-thin sectioning. Thin 1-µm sections were mounted on copper mesh grids, stained with uranyl acetate and lead citrate, and examined on a JEOL 1200 EXII electron microscope. 30-50 micrographs were taken randomly from each sample at primary magnification × 10,000.

### Electrophysiological Studies.

Whole cell currents were monitored from Sertoli cells, CFTR^{-/-} and WT renal epithelial cells with an RK 400 amplifier (Biologic, Münster, Germany) as previously described (40). The bath (extracellular) solution consisted of 140 mM NMDGCl (N-methyl-D-glucamine chloride), 5 mM D-Glucose, 1 mM CaCl₂, and 10 mM HEPES, pH 7.4. Patch pipette (intracellular) solution contained 140 mM NMDGCl, 5 mM D-Glucose, 5 mM EGTA, 5 mM MgATP, and 10 mM HEPES, pH 7.4. Osmolarity of both solutions was adjusted to 280 mOSM by adding mannitol. These solutions were designed to study Cl⁻ currents because Cl⁻ is the sole permeant ion in these conditions and to prevent Ca²⁺ activated Cl⁻ currents by the addition of EGTA in the patch solution. After establishment of whole cell configuration, cells were stimulated by the addition of Lindane (50 µM), FSK (10 µM), vehicle (Me₂S0 or ethanol) in the absence or the presence of NPPB (0.1mM), DIDS (4,4'-diisothiocyanatostilbene-2,2'-disulfonic acid, 1 mM, Sigma), glibenclamide (0.1mM, Sigma) or PD98059 (10 µM) in the bath solution. The cell membrane potential was clamped at -50mV and 400 ms pulses were applied from -100 to +120 mV with increments of 20 mV every 2 s. To generate whole cell current-voltage (*I-V*) relationship, membrane currents resulting from voltage stimuli were recorded for at least 3 min and analysed using an IBM-AT-compatible computer equipped with Digidata 1200 interface (Axon Instruments) and pCLAMP software (versions 5.51 and 6.0, Axon Instruments), respectively. *Statistics*-Results are expressed as mean ± S.E. of *n* observations using the Student's *t* test.

### Immunofluorescence staining.

Sertoli cells seeded on glass coverslips were transiently transfected with EGFP-Rab7 or MEK₁⁺-HA expression plasmids using Fugene 6 reagent (Roche), washed and allowed to recover overnight before addition of Lindane (50 µM). 24 h later, indirect immunofluorescence was performed using antibodies against HA (UBI) or specific markers of lysosomes (LAMP2, Transduction laboratories) as described (58). To determine the pH of the Lindane-induced vacuoles, living cells were stained with acridine-orange (5 µg/ml, Sigma), a membrane-permeable weak base whose fluorescence shifts from green at neutral pH to red in acidic compartments (*59*). 10 min later, cells were washed and directly observed under a confocal microscope. Pictures were taken with a 63x magnification lens using a confocal laser-scanning microscope (Leica) fitted with a 488 or 543 nm krypton/argon laser allowing simultaneous analysis of the fluorescein and rhodamine chromophores.

### Western blotting.

Cell lysates were prepared in NP4O/Brij lysis buffer and analyzed by Western blotting as previously described (58) with antibodies that recognize the phosphorylated active forms of ERK, Jun kinases, or p38 (Cell Signaling). After stripping, equal loadings of proteins were verified by reprobing the blots with anti-ERK1 (Santa Cruz Biotechnology).

### Results and Discussion:

We focused our attention on a widely used pesticide Lindane (γ-hexachlorocyclohexane) and we show that it is a powerful activator of autophagy (Fig. 1). Indeed, using the 42GPA9 Sertoli cell-line, we show that addition of Lindane (50 µM) causes the rapid formation of large vacuoles within 8h of treatment (Fig. 1A, *insert*).
Electron microscopy analyses reveal that these structures were autophagic vacuoles (Fig. 1B) as they were *i) double membrane-enclosed* vesicles, *ii*) containing *fragments of organelles* and *iii)* dramatically reduced by the *autophagy inhibitor 3 methyladenine* (3 MA, 30 mM, (28) (Fig. 1C). Interestingly, the ultrastructure of organelles were identical to control condition; without any signs of degeneration. In particular, the number and the shape of mitochondria appeared unaffected [Fig. 1E shows mitochondria (indicated by M) with clear cristae at proximity of autophagosomes]. Many autophagosomes were formed by the sequestration of part of cytoplasm by a double membrane (Fig. 1 B-E). During the course of the experiment, both the number and size of vacuoles increased, and many of them were single membrane bound autolysosomes containing degraded content. Even during the late stages, vacuolated cells did not stain with propidium iodide and the nuclei exhibited normal morphology with finely dispersed chromatin and a large nucleolus. Consistently, little if any dead cells together with the observation of reversibility demonstrated that Lindane did not induce cell vacuolation by promoting cell death. Furthermore, longer exposure to this pesticide for four days was not associated with cell lysis but rather with coalescence of the vacuoles. Importantly, the absence of several apoptotic morphological and biochemical features such as caspase 3 activation, PARP cleavage; chromatin condensation, nuclear pyknosis and mitochondria degeneration indicates that Lindane-induced autophagy did not utilize common machinery with apoptosis. This is a fundamental point which distinguishes Lindane from most of the other autophagic inducers (such as ischemia, pathogens, heat, or oxidation...*(60)*). Therefore, Lindane-induced vacuolation provides a working model for delineating the specific molecular events that control autophagy.

All autophagic stages were observed within Lindane-treated cells from initial lamellar structures sequestrating organelles to autophagosomes in close vicinity with late endosomes and lysosomes; likely in the initial stage of fusion (Fig. 1F). Along the autophagic pathway, maturation of autophagosomes into autolysosomes involves fusions with late endosomal/lysosomal compartments and acidification by the Vacuolar-type H⁺ ATPase (V-ATPase) (2, 8). Consistently, confocal microscopy indicated that Rab7 and LAMP2, two markers of late endosomes and lysosomes respectively, surrounded the vacuoles (Fig. 2A). Moreover, Lindane-induced vacuoles were acidic (Fig. 2A) and addition of bafilomycin A1 (100 nM, BafA1), a specific V-ATPase inhibitor, prevented their formation (Fig. 2B). In agreement with the involvement of anion channels in V-ATPase activity (*61*), pre-treatment with the Cl⁻ channel blocker NPPB (25 µM) abrogated Lindane-induced vacuolation (Fig. 2B). Interestingly, addition of BafA1 or NPPB collapsed Lindane-preformed vacuoles. Therefore, both V-ATPase and Cl⁻ channel activities were required for Lindane-induced formation and maintenance of large autolysosomes.

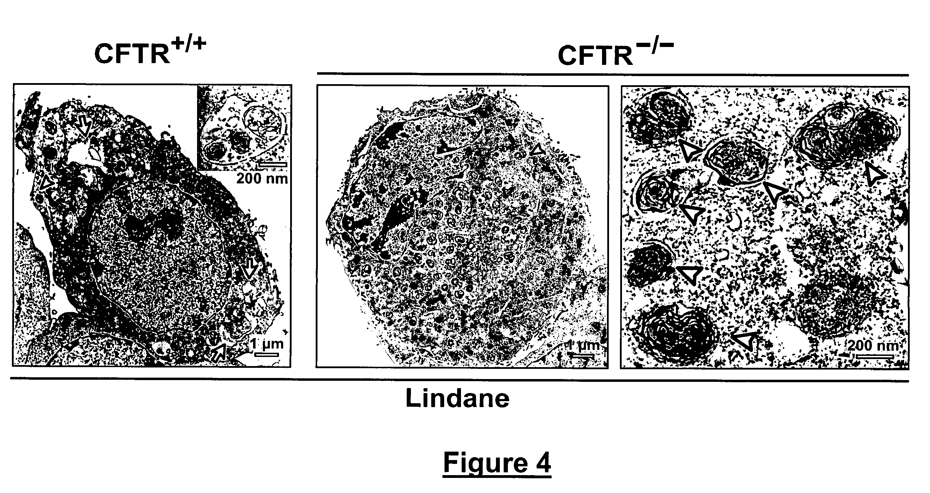

Several Cl⁻ channels are present in the plasma membrane and in intracellular organelles together with V-ATPase (61). Whole-cell recording patch-clamp experiments revealed that Lindane increased within minutes Cl⁻ conductance across plasma membrane of Sertoli cells (Fig. 3A), reported to express CFTR and CIC Cl⁻ channels (62, 63). By several criteria, we established that Lindane activated CFTR currents. Indeed, Lindane-induced currents displayed similarly to FSK-activated CFTR i) a *linear* current-voltage relationship with a reversal potential at ~0 mV (Fig. 3B); *ii*) a *sensitivity* to NPPB, glibenclamide but not to external DIDS (Fig. 3A); and *iii)* a typical Br⁻≥ Cl⁻>>I⁻ *anion selectivity* sequence. Demonstration that CFTR channel accounted for Lindane-induced Cl⁻ conductance was obtained by the induction of Cl⁻ currents by Lindane in CFTR^{+/+} but not in CFTR^{-/-} renal distal cells (Fig. 3C). Of particular interest, CFTR was redistributed from plasma membrane to vacuoles in Lindane-treated cells (Fig. 3D). Importantly, the ability of Lindane to promote autophagic vacuolation could be generalized to renal cells, provided CFTR was expressed (Fig. 4). Strikingly, the loss of CFTR expression led to an altered Lindane-induced response with dramatic accumulation of lamellar structures and little if any large autophagic vacuoles (Fig. 4). Taken together, these pharmacological and genetic approaches strongly suggest that the maturation of autophagic vacuoles by Lindane was dependent on CFTR activation.

The emerging issue is therefore how Lindane could activate CFTR Cl⁻ currents. One possibility would be that Lindane directly activates CFTR, but this appears unlikely because it required minutes instead of seconds to increase Cl⁻ conductance. Alternatively, Lindane could indirectly regulate CFTR by activating signaling pathways, as suggested by the critical role played by PKA in CFTR opening (64). Consistently, a dramatic phosphorylation of CREB (cAMP binding response element), as readout of PKA activity was observed upon Lindane treatment. However, PKA inhibition by H89 failed to counteract Lindane-induced CREB phosphorylation, indicating that Lindane did not activate PKA. To further explore this hypothesis, we tested the effect of Lindane on the renal proximal cell line (PCT1). Indeed, Philippe Poujeol's group has demonstrated that this renal cell-type presents the advantage of expressing a splice variant of CFTR deleted of exon10 (CFTR Δex10). The loss of exon10 leaves the open reading frame intact, suggesting that this transcript could be translated. But the encoded product lacks part of the R domain with the major phosphorylations sites for protein kinase A. Consistently; we found that Forskolin failed to activate a Cl- current in PCT cells (Fig. 11). Of particular interest, we provide several lines of evidence that the CFTR Δex10 channels remained functional in response to Lindane in PCT cells. This evidence includes the following: i) Lindane induced a Cl- current with a *linear* current-voltage relationship with a reversal potential at ~0 mV, similar to that of cAMP-activated Cl- channels in native DCT. *ii*) a *sensitivity* to NPPB, glibenclamide but not to external DIDS; and 3) a typical Br⁻≥ Cl⁻ >>I⁻ *anion selectivity* sequence. *iii)*, the total inhibition of this Lindane-induced Cl-current in CFTR^{-/-} PCT cells demonstrated that it corresponded to CFTR. These finding provide the first evidence that an agonist, *e*.*g*. Lindane, could activate CFTR Cl- current independently of PKA, raising the promise for new strategies using Lindane to restore Cl-conductance in cystic fibrosis patients with CFTR variants on PKA sites.

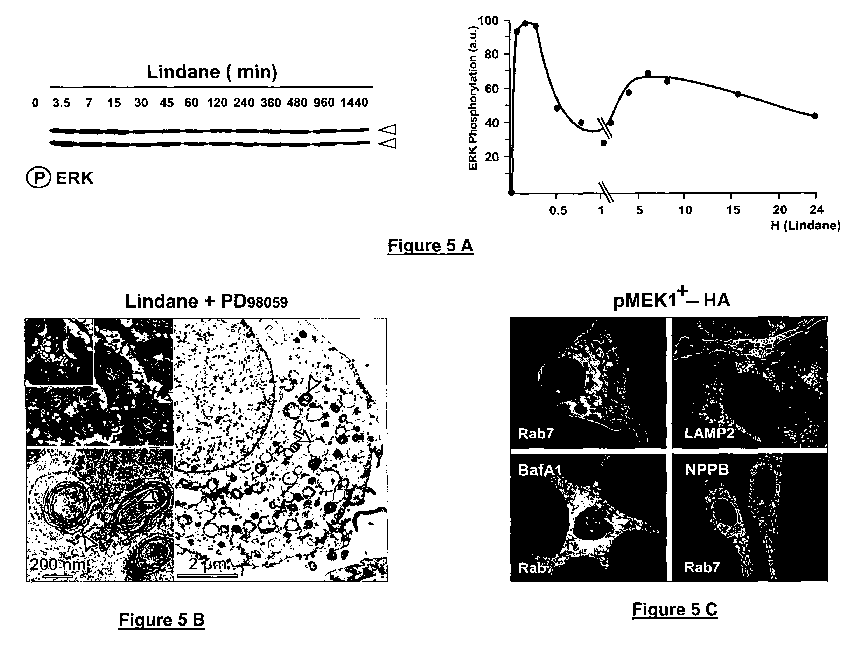

MAPKs ERK1/2 are candidates known to phosphorylate CREB, activate ion channels (*65-67*) and autophagy (*10*). Treatment of Sertoli cells with Lindane induced a rapid and sustained activation of both p42^{ERK2} and p44^{ERK1} (Fig. 5A). By contrast, the stress-activated kinases Jun kinases or p38 were not activated in Lindane-treated cells (38), suggesting a specific role for ERK. Consistently, pre-treatment with the MEK_{1/2} inhibitor

PD₉₈₀₅₉ (10 µM) which drastically reduced the ability of Lindane to activate ERK, abrogated Lindane-induced vacuolation (Fig. 5B). Ultrastructural analysis evidenced a dramatic accumulation of lamellar structures containing parts of cytoplasm, establishing as for CFTR an essential role of *ERK in the autophagosome maturation stage* (Fig. 5B).

By the use of a dominant MEK1⁺ construct, we provide the first evidence that activation of the ERK pathway was sufficient to trigger swelling of rab7/LAMP2 containing vacuoles (Fig. 5C). Consistently, the oncogenic activation of ras (ras^{V12}), the upstream activator of ERK, induces autophagic-vacuolation and -degradation in glioma, gastric or intestinal cancer cell-lines *(68, 69).* However, these studies have not addressed whether ras^{V12-}induced autophagy is dependent on ERK. In addition, expression of MEK1⁺ in transgenic mice has been reported to result in lens and muscular cell vacuolation *(70, 71);* but the nature of enlarged compartment remains unknown. These data created an apparent paradox in that growth factors also stimulate ERK and yet have no detectable effect on autophagy (69). An attractive possibility to explain this discrepancy might be to consider that the cell response depends critically of the nature, strength and duration of MAPK pathways activated (*39*). Interestingly while growth factors trigger transient ERK activity with multiple pathways, we have shown that Lindane, as did MEK1⁺ (*72*), induced a *sustained* and *specific* ERK activation for more than 24h (Fig. 5A). In this regard, evidence that autophagy plays an essential role during cell differentiation (*4, 5*), a process induced by sustained ERK activation (*39*), is of great interest. Altogether, our results point to the new notion that *autophagic vacuolation* is not merely an adaptive cellular response to an environmental stress but rather the exaggerated consequence of sustained and *specific ERK activation.*

We therefore examined the order of function between ERK and CFTR. ERK was still stimulated by Lindane in NPPB pre-treated cells or in CFTR^{-/-} cells, indicating a CFTR-independent ERK activation (Fig. 6A). By contrast, CFTR Cl- conductance from Lindane-induced cells was dramatically reduced in the presence of PD98059 (Fig. 6B), providing the first evidence that ERK pathway could participate in CFTR channel activation.

Taken together, these findings strongly support a novel mechanism by which ERK pathway controls autophagy at the maturation stage mainly through CFTR activation (Fig. 7). The localization of CFTR to autophagic vacuoles led us to propose that ERK may target in the autophagosomal compartments a CFTR-like Cl⁻ conductance, ensuing V-ATPase activation, acidification, and digestion by acid proteases of the sequestered cargo into nutrients. This prompted us to challenge the responses of CFTR^{-/-} cells to nutrient deprivation, a physiological inducer of autophagy. In such a model, it might be expected that the loss of CFTR would sensitize cell to starvation-induced death, as a consequence of this autophagic defect. In agreement with our model, in response to starvation CFTR^{-/-} cells activated ERK at the same level as did CFTR^{+/+} cells (Fig. 8A), exhibited lamellar bodies but failed to form autolysosomes (Fig. 8B). Of particular interest, the starved CFTR^{-/-} cells underwent massive autophagic death within 3-6 hours that was rescued by addition of amino acids, the end product of autophagy (Fig. 8C). Consistently, CFTR^{+/+} cells which effectively activated autophagy to autolysosomes survived starvation at least 24 hours (Fig. 8B and 8C). In agreement with the above-demonstrated control of autophagosome maturation by ERK/CFTR pathway, inhibition of ERK or Cl⁻ Channel activity committed the CFTR^{+/+} cells to starvation-induced autophagic death (Fig. 8D). Similar autophagic defects (lamellar bodies and autophagic death) have been observed when V-ATPase is inhibited *(30, 73*), further strengthening our model. These findings point out to the important notion that the activation of CFTR is absolutely required for autophagy, thereby enabling cell survival under deprivation stress.

There is increasing evidence linking CFTR to autophagy. Indeed, it has been reported that AMP-activated protein kinase which inactivates CFTR (74) counteracts the autophagy process (*75*, *76*). Notably, PIST a recently identified ligand of CFTR (also called CAL for CFTR associated ligand (77) has emerged as a regulator of autophagic neurodegeneration by linking the *activated* δ2 glutamate channel receptor to Beclinl (78), a major determinant in autophagy dynamics. A role for PIST in CFTR trafficking has been invoked by the redistribution of CFTR from the plasma membrane to unidentified intracellular compartments in PIST-overexpressing cells (77). A major goal for future studies will be to define the precise mechanism(s) by which the ERK pathway activates CFTR and thereby autophagy. One possibility would be that ERK might directly phosphorylate CFTR, as suggested by the presence within CFTR sequence of potential ERK-docking ("D box": ²⁵RKGYRQRLELSDIYQIPSVD⁴⁴; minimal FXP/F motif: ¹²⁹⁴FIF¹²⁹⁶, (*72, 79, 80*) and -phosphorylation ["(S/T)P" at T⁴³⁸, S⁵⁷³, T⁷¹⁷ and S¹²³⁵] sites (Fig. 6). Alternatively, ERK might phosphorylate proteins that interact with and regulate CFTR. Whether ERK controls by activating CFTR, the assembly of CFTR/PIST/Beclin1 complex remains to be determined.

Most importantly, we demonstrate that two cellular consequences of CFTR loss are the dramatic *accumulation of lamellar bodies* and the promotion of *starvation-induced death.* These findings not only identify CFTR as a critical regulator of autophagy but also open the critical issue of whether the *loss-of- this function* might contribute to the pathogenesis of cystic fibrosis, the most common inherited disease. Along this line, it has been recognized that CF could not be explained solely by the loss of CFTR Cl⁻ channel activity at the plasma membrane as the CF patients maintain normal Cl⁻ concentrations in their lung parenchyma (24). Strikingly, this defective autophagy hypothesis may help clarify many of clinical CF features:
*i)* a massive epithelial cell death is indeed responsible for the lethal CF *respiratory distress;*
*ii)* CF patients are particularly sensitive to chronic *bacterial infections* which are well-established inducers of autophagy (*3*, *22*);
*iii) malnutrition* which is a consequence of CF gastrointestinal dysfunction is a bad prognostic factor for patient survival (*22*, *81*);
*iv) concentrated mucous* which burden the CF lung could be ascribed in part to the accumulation of lamellar autophagic bodies that function as the secretory granules of surfactant (82, 83).

Therefore, the discovery of the critical role of CFTR in autophagy points out new attractive avenues for exploring the elusive cystic fibrosis pathogenesis raising the rationale to fight this disease.

### EXAMPLE 2: Inhibiting p38 induces autophagy

We compared several xenobiotics known to elicit *in vivo* different responses: DDT and biphenol A are reported to promote Sertoli cell vacuolation whereas Pentachlorophenol does not *(84)*. Consistently, we showed *in vitro* that DDT (1.8 µM) and biphenol (Bϕ, 90 µM) but not Pentachlorophenol (PCP, 15 µM) induced vacuolation in Sertoli 42GPA9 cells (Fig. 9A).

To gain insight into the underlying molecular mechanisms, we examined the possibility that this cell response is due to the activation of signaling pathways, as suggested by the above demonstrated regulation of autophagy by the MAP kinases ERK. As indicated in Fig. 9B, analysis of cell lysates by anti-phospho-ERK western blotting indicated that Lindane, DDT and Bϕ induced the activation of both p42^{ERK2} and p44^{ERK1} in Sertoli cells, suggesting that ERK activation may contribute to cell vacuolation. However, no vacuolation was observed in response to Pentachlorophenol (PCP, 15 µM) although it was strong activator of ERK1/2. Interestingly, PCP concomitantly activated p38 in contrast to Lindane, DDT and Bϕ. Given that activation of p38 was reported to antagonize the ERK pathway (*85*, *86*) and starvation-induced autophagy (*87*); we consider the hypothesis that chemicals may not only trigger ERK activation but also concurrently suppress p38 activity. The inactivation of p38 and/or the activation of ERK may be critical for autophagic vacuolation.

This prompted us to investigate the possible involvement of p38 inhibition in vacuolation by using SB203580 (10 µM), a specific p38 inhibitor. In agreement with our hypothesis, we found that SB203580 promoted by itself the formation of large Rab7⁺ acidic vacuoles in Sertoli cells, reminiscent of those seen in Lindane-exposed cells (Fig. 10).

Numerous inhibitors of p38 have demonstrated efficacy in animal models of arthritic disease and are currently in phase II clinical trials for rheumatoid arthritis. However, a significant problem faced by pharmaceutical companies is the unexpected toxicities of these small compounds in the later stages of development. Side effects on autophagic pathway should be therefore taken into consideration when molecules inhibiting p38 will be designed for therapy of rheumatoid arthritis.

### EXAMPLE 3: Biotest to monitor autophagy

Although worldwide concerns have emerged about increasing number of environmental factors that display adverse effects on human health, an *in vitro* bioassay that rapidly screens the deleterious effects of chemicals is still lacking.

Compelling epidemiologic studies have reported an decrease in male infertility and a rising incidence of malformations and several malignancies (breast, uterine, testicular and liver cancers, leukaemia...) in industrialized countries of Europe and North America (*88*, 89). Because of this consistent trend, a chronic exposure to environmental chemicals has been suspected (90). Considerable efforts have been therefore devoted towards the evaluation of toxic and carcinogenic effects of these compounds (*91*). It turns out that widely used pesticides are lipophilic compounds that accumulate in human tissues (testes, adipose tissue...) (92). There, their toxicities persist for years and are able to promote malformations, decreased fertility and several cancers in exposed rodents (93, 94). As a result, risk-assessment of chemicals became an important issue of public health policy.

Most of studies carried so far have used laboratory animals as model for evaluating the potential toxicity of chemicals. Histological analyses have revealed that one of the earliest damages induced by toxicants is the selective vacuolation of Sertoli cells, before the onset of germ cell apoptosis and loss (84, 94). Within the testis, Sertoli cells support spermatogenesis by controlling germ cell growth and differentiation and consequently, alterations of Sertoli cell functions are expected to contribute to infertility or testicular cancer. Therefore, vacuolation of Sertoli cells could be used as an early and important marker of chemical-induced deleterious effects on the male reproductive system. However, development of such application requires a better knowledge of pesticide effects at both the cellular and molecular levels. Indeed, we are ignorant of the exact nature of the response: direct or indirect; given the complex interdependence of testicular cells. Moreover, neither the nature of enlarged compartments nor the underlying molecular mechanisms has been studied.

The mouse Sertoli cell line 42GPA9 offers a promising and convenient model for investigating the effects of toxicants. Indeed, the 42GPA9 cells retain several morphological and biochemical features of differentiated adult Sertoli cells *in vivo.* For example, they exhibit tight and gap junctions and they also produce Transferrin, sulfated glycoprotein-2, and Stem Cell Factor (53).

Using this cell line, we found that a widely used carcinogen pesticide, Lindane *directly* promoted vacuolation of Sertoli cells (Fig. 1). This injury started at 9h, was maximal at 24-48h of treatment and is reversible upon Lindane removal. By Electron and immunofluorescent microscopy analyses, we demonstrated that these structures were autolysosomes, containing fragments of organelles, and labeled by rab7 and LAMP2, two markers of late endosomes and Lysosomes respectively (Fig. 2A). Consistently, we show that these autophagic vacuoles were acidic as they take up membrane permeable weak bases such as the dye neutral red and Acridine orange (Fig. 2A).

A similar Sertoli cell damage was observed with other common xenobiotics such as DDT and bisphenol A (Fig. 9). Moreover, the Lindane-induced autophagic damages we readily observed upon a light microscope in Sertoli cells (Fig 1) were also detected in renal cells, but only by time-consuming electronic microscopy (Fig 4). Therefore, the 42GPA9 Sertoli cell line could serve as a useful cellular model for testing chemicals on autophagy; a process that occurs in all eukaryotic cells (*1*).

Altogether, these findings illustrate that *in vitro* vacuolation of Sertoli 42GPA9 cells can be used as a bioassay in toxicological studies for screening the deleterious effect of chemicals on the autophagy pathway and the male reproductive system as this method fulfills the following criteria:
1) rapidity: requires hours *versus* weeks and months for animal studies;
2) earliest marker: precedes other widely used markers such as the expression of metabolizing enzymes;
3) low cost: vacuoles can be readily observed by phase contrast microscopy;
4) relevant of cellular damages observed in exposed animals;
5) sensitive; and measurable: allows quantitative comparison of several chemicals by uptake of Acridine orange or neutral red;
6) provides valuable information for predicting the deleterious effects of chemical on human health;
7) could be used for the rapid detection of toxic compounds in biological fluids, in drinking water, etc.

### BIBLIOGRAPHIC REFERENCES :

- 1.: D. J. Klionsky, S. D. Emr, *Science* **290**, 1717-1721 (2000).
- 2.: J. Kim, D. J. Klionsky, *Annu. Rev. Biochem.* **69**, 303-342 (2000).
- 3.: K. Kirkegaard, M. P. Taylor, W. T. Jackson, *Nat. Rev. Microbiol*. **2,** 301-314 (2004).
- 4.: A. Melendez *et al*., *Science* **301,** 1387-1391 (2003).
- 5.: Z. Yue, S. Jin, C. Yang, A. J. Levine, N. Heintz, *Proc. Natl. Acad Sci. U.S.A*. ***100,*** 15077-15082 (2003).
- 6.: X. *Qu et al., J. Clin. Invest*. **112,** 1809-1820 (2003).
- 7.: A. L. Edinger, C. B. Thompson, *Cancer Cell* 4, 422-424 (2003).
- 8.: Y. Ohsumi, *Nat. Rev. Mol. Cell Biol*. **2**, 211-216 (2001).
- 9.: A. Petiot, S. Pattingre, S. Arico, D. Meley, P. Codogno, *Cell Struct. Funct. 27,* 431-441 (2002).
- 10.: E. Ogier-Denis, S. Pattingre, J. El Benna, P. Codogno, *J. Biol. Chem*. **275**, 39090-39095 (2000).
- 11.: J. R. Riordan, J. M. Rommens, B. S. Kerem, e. al., *Science* **245,** 1066-1073 (1989).
- 12.: J. M. Rommens, M. C. Iannuzzi, B. S. Kerem, e. al., *Science* **245**, 1059-1065 (1989).
- 13.: J. Zielenski *et al., Genomics* **10,** 214-228 (1991).
- 14.: D. N. Sheppard, M. J. Welsh, *Physiol. Rev.* **79,** 23-45 (1999).
- 15.: E. M. Schwiebert, D. J. Benos, M. E. Egan, M. J. Stutts, W. B. Guggino, *Physiol. Rev.* **79,** S145-S166 (1999).
- 16.: G. B. *Pier et al., Science* **271,** 64-67 (1996).
- 17.: G. B. Pier, M. Grout, T. S. Zaidi, *Proc. Natl. Acad. Sci. U. S. A.* **94,** 12088-12093 (1997).
- 18.: S. A. Lahousse, E. G. Stopa, A. E. Mulberg, S. M. de la Monte, *J*. *Alzheimers Dis.* 5, 455-462 (2003).
- 19.: A. E. Mulberg *et al., J. Clin. Invest.* **96**, 646-652 (1995).
- 20.: G. Nagel, T. C. Hwang, K. L. Nastiuk, A. C. Nairn, D. C. Gadsby, *Nature* **360,** 81-84 (1992).
- 21.: T. V. McDonald, P. T. Nghiem, P. Gardner, C. L. Martens, *J*. *Biol. Chem*. **15**, 3242-3248 (1992).
- 22.: F. Ratjen, G. Doring, *Lancet* **361**, 681-689 (2003).
- 23.: K. E. Barrett, S. J. Keely, *Annu. Rev. Physiol.* **62**, 535-572 (2000).
- 24.: A. S. Verkman, Y. Song, J. R. Thiagarajah, *Am. J. Physiol. Cell Physiol.* **284**, C2-15 (2003).
- 25.: E. H. Oppenheimer, J. R. Esterly, *Perspect. Pediatr. Pathol.* **2**, 241-278 (1975).
- 26.: C. W. Chow, L. I. Landau, L. M. Taussig, *Eur. J. Pediatr*. **139**, 240-243 (1982).
- 27.: N. Mizushima, *Int. J. Biochem. Cell Biol*. **36,** 2491-2502 (2004).
- 28.: P. O. Seglen, P. B. Gordon, *Proc. Natl. Acad. Sci. USA* **79,** 1889-1892 (1982).
- 29.: L. Yu *et al., Science* **304,** 1500-1502 (2004).
- 30.: A. Yamamoto *et al., Cell Struct. Funct.* **23,** 33-42 (1998).
- 31.: T. Kirisako *et al., J. Cell Biol*. **147**, 435-446 (1999).
- 32.: Y. *Kabeya et al., EMBO J.* **19**, 5720-5728 (2000).
- 33.: Y. *Kabeya et al., J. Cell Sci*. **117**, 2805-2812 (2004).
- 34.: H. *He et al., J. Biol. Chem.* **278**, 29278-29287 (2003).
- 35.: N. Mizushima, A. Yamamoto, M. Matsui, T. Yoshimori, Y. Ohsumi, *Mol. Biol. Cell.* **15**, 1101-1111 (2004).
- 36.: F. *Mendes et al., J. Cyst. Fibros.* **3**, 69-72 (2004).
- 37.: M. Benharouga *et al., J. Biol. Chem.* **278**, 22079-22089 (2003).
- 38.: B. *Mograbi et al., Carcinogenesis* **24**, 1415-1423 (2003).
- 39.: C. J. Marshall, *Cell***80,** *179-185* (1995).
- 40.: H. *Barriere et al., Am. J. Physiol. Renal. Physiol*. **284,** F796-F811 (2003).
- 41.: D. N. Sheppard, M. J. Welsh, *Physiol Rev.* **79,** S23-S45 (1999).
- 42.: N. Sewald, H.-D. Jakubke, Peptides: Chemistry and Biology, *Wiley-VCH Verlag GmbH, Weinheim* (2002).
- 43.: K. Müller *et al*., Perspectives in Medicinal Chemistry, in B. Testa, E. Kyburz, W. Fuhrer, R. Giger, Eds. (Verlag Helv. Chim. Acta, Basel, 1993) pp. 513-533.
- 44.: R. Aurora, G. D. Rose, *Protein Sci.* 7, 21-38 (1998).
- 45.: D. Obrecht, M. Altorfer, J. A. Robinson, *Adv. Med. Chem.* **4,** 1-68 (1999).
- 46.: W. Maison, E. Arce, P. Renold, R. J. Kennedy, D. S. Kemp, *J. Am. Chem. Soc.* **123**, 10245-10254 (2001).
- 47.: F. Formaggio *et al., J. Peptide Sci.* **9**, 461-466 (2003).
- 48.: J. S. Nowick, J. O. Brower, *J. Am. Chem. Soc*. **125,** 876-877 (2003).
- 49.: C. *Lebrand et al., EMBO J.* **21,** 1289-1300 (2002).
- 50.: J. Milanini, F. Vinals, J. Pouyssegur, G. Pagès, *J. Biol. Chem*. **273,** 18165-18172 (1998).
- 51.: V. Paquis-Flucklinger *et al*., *Oncogene* **8**, 2087-2094 (1993).
- 52.: Z. Y. Zhu *et al*., *J Virol*. **51,** 170-180 (1984).
- 53.: V. Bourdon, A. Lablack, P. Abbe, D. Segretain, G. Pointis, *Biol. Reprod.* **58**, 591-599 (1998).
- 54.: A. Steinberger, A. Jakubowiak, in *The Sertoli cell* L. D. Russell, M. D. Griswold, Eds. (Cache River Press, Clearwater, Fla, 1993) pp. 155-180.
- 55.: I. *Rubera et al., Am. J. Physiol*. **276,** F104-F121 (1999).
- 56.: e. Chambard, *J. Cell Biol*. **91,** 157-166 (1981).
- 57.: G. Le'Negrate, E. Selva, P. Auberger, B. Rossi, P. Hofman, *J. Cell Biol*. **150,** 1479-1488 (2000).
- 58.: B. Mograbi *et al., Mol. Cell. Biol*. **21,** 6719-6730 (2001).
- 59.: C. Millot, J. M. Millot, H. Morjani, A. Desplaces, M. Manfait, *J Histochem Cytochem.* **45**, 1255-1264 (1997).
- 60.: D. Gozuacik, A. Kimchi, *Oncogene* **23,** 2891-2906 (2004).
- 61.: T. Nishi, M. Forgac, *Nat. Rev. Mol. CelI Biol*. **3**, 94-103 (2002).
- 62.: F. R. Boockfor, R. A. Morris, D. C. DeSimone, D. M. Hunt, K. B. Walsh, *Am. J. Physiol*. **274,** C922-930 (1998).
- 63.: C. Auzanneau, V. Thoreau, A. Kitzis, F. Becq, *J. Biol. Chem.* **278**, 19230-19236 (2003).
- 64.: D. *Dahan et al., J. Cell Sci.* **443,** S92-S96 (2001).
- 65.: V. Crepel, W. Panenka, M. E. Kelly, B. A. MacVicar, *J Neurosci.* **18**, 1196-1206 (1998).
- 66.: S. Giovannardi *et al., J Biol. Chem.* **277,** 12158-12163 (2002).
- 67.: H. *Shi et al., J Biol. Chem.* **277**, 13539-13547 (2002).
- 68.: S. *Chi et al., Oncogene* **18**, 2281-2290 (1999).
- 69.: S. Pattingre, C. Bauvy, P. Codogno, *J. Biol. Chem.* **278,** 16667-16674 (2003).
- 70.: X. *Gong et al., Invest. Ophthalmol. Vis. Sci.* **42**, 539-548 (2001).
- 71.: S. *Nakano et al., Neurology* **56,** 87-93 (2001).
- 72.: G. *Pearson et al., Endocr. Rev.* **22,** 153-183 (2001).
- 73.: T. Kanzawa, Y. Kondo, H. Ito, S. Kondo, I. Germano, *Cancer Res*. **63,** 2103-2108 (2003).
- 74.: K. R. Hallows, J. E. McCane, B. E. Kemp, L. A. Witters, J. K. Foskett, *J. Biol*. *Chem.* **278**, 998-1004 (2003).
- 75.: H. R. Samari, S. P.O., *J. Biol. Chem*. **273**, 23758-23763 (1998).
- 76.: Z. Wang, W. A. Wilson, M. A. Fujino, P. J. Roach, *Mol. Cell. Biol*. **21,** 5742-5752 (2001).
- 77.: J. *Cheng et al., J. Biol. Chem*. **277**, 3520-3529 (2002).
- 78.: Z. Yue *et al., Neuron* **35,** 921-933 (2002).
- 79.: D. Jacobs, D. Glossip, H. Xing, A. J. Muslin, K. Kornfeld, *Genes Dev*. **13,** 163-175 ( 1999).
- 80.: D. Barsyte-Lovejoy, A. Galanis, A. D. Sharrocks, *J Biol Chem.* **277**, 9896-9903 (2002).
- 81.: R. Kraemer, A. Rudeberg, B. Hadom, E. Rossi, *Acta Paediatr. Scand.* **67**, 33-37 ( 1978).
- 82.: M. *Hariri et al., Mol. Biol. Cell*. **11,** 255-268 (2000).
- 83.: M. Larbig *et al., Pathobiology* **70,** 89-97 (2002-2003).
- 84.: K. Boekelheide, in *The Sertoli cells* L. D. Russell, M. D. Griswold, Eds. (Cache River Press, 1993) pp. 551-575.
- 85.: Z. Xia, M. Dickens, J. Raingeaud, R. J. Davis, M. E. Greenberg, *Science* **270,** 1326-1331 (1995).
- 86.: S. D. Zatechka, M. F. Lou, *Exp Eye Res* **75,** 177-192 (2002).
- 87.: S. vom *Dahl et al., Biochem. J*. **354,** 31-36 (2001).
- 88.: A. Ekbom, O. Akre, *APMIS*. **106**, 225-229; discussion 229-231 (1998).
- 89.: J. M. Mckiernan, E. T. Goluboff, G. L. Liberson, R. Golden, H. Fisch, *J. Urol*. **162**, 361-363 (1999).
- 90.: N. E. Skakkebaek, *Horm. Res.* **57**, 43 (2002).
- 91.: J. Dich, S. H. Zahm, A. Hanberg, H.-O. Adami, *Cancer Causes Control* **8,** 420-443 (1997).
- 92.: A. K. Prasad, N. Pant, S. C. Srivastava, R. Kumar, S. P. Srivastava, *Hum. Exp. Toxicol.* **14**, 484-488 (1995).
- 93.: IARC, IARC, Ed. (Lyon, 1990), vol. 100, pp. 352.
- 94.: P. R. Dalsenter, A. S. Faqi, J. Webb, H. J. Merker, I. Chahoud, *Hum. Exp. Toxicol*. **15**, 406-410 (1996).

## Claims

1. Process for identifying a compound useful for the manufacture of a drug intended for the preventive and/or palliative and/or therapeutic treatment of cystic fibrosis,
**characterized in that** it comprises providing a candidate compound, and
a) contacting said candidate compound with a eukaryotic CFTR+/+ cell, and determining whether said candidate compound induces and/or stimulates at least one among the following features:
a1) the formation of autophagic vacuoles in said eukaryotic CFTR +/+ cell,
a2) the formation of CFTR-expressing vacuoles within the cytoplasmic compartment of said eukaryotic CFTR +/+ cell,
a3) the delocalization of CFTR proteins from the plasma membrane to the cytoplasmic compartment of said eukaryotic CFTR +/+ cell,
and/or
b) determining whether said candidate compound is capable of phosphorylating a CFTR protein, and/or of stimulating such a phosphorylation, and/or of inducing such a phosphorylation, and/or
c) contacting said candidate compound with a growth factor-deprived and nutrient-deprived eukaryotic CFTR-/- cell, and determining whether said candidate compound prevents or delays the autophagic death of said cell,
where a positive determination at said step a) and/or b) and/or c) identifies said compound as being useful for the manufacture of a drug intended for the preventive and/or palliative and/or therapeutic treatment of cystic fibrosis.

2. Process according to claim 1, **characterized in that** the autophagic vacuoles of said step a1) comprise autophagosomes and/or autolysosomes.

3. Process according to claim 1, **characterized in that** said step b) comprises determining whether said candidate compound is capable of phosphorylating a CFTR protein on at least one ERK phosphorylation site, and/or of stimulating such a phosphorylation, and/or of inducing such a phosphorylation.

4. Process according to claim 3, **characterized in that** said CFTR protein is a human CFTR protein, and said at least one ERK phosphorylation site is selected from the group consisting of S4, T438, S573, T717, S1235.

5. Process according to any one of claims 1-4, **characterized in that** it further comprises determining whether said candidate compound is capable of inducing and/or stimulating a CFTR-regulated anionic and/or cationic current in a eukaryotic CFTR+/+ cell.

6. Process according to any one of claims 1-5, **characterized in that** said eukaryotic cell is an epithelial cell.

7. Process according to any one of claims 1-6, **characterized in that** said candidate compound is an activated kinase, or a kinase activator, or a kinase inhibitor.

8. Process according to claim 7, **characterized in that** said candidate compound is an activator of the MEK/ERK/CFTR pathway.

9. Process according to claim 7, **characterized in that** said candidate compound is a p38 inhibitor.

10. Process according to any one of claims 1-9, **characterized in that** it further comprises selecting those candidate compounds which stimulate cell differentiation, and/or discarding those candidate compounds which stimulate cell proliferation.

11. Process according to any one of claims 1-10, **characterized in that** said compound that is useful for the manufacture of a drug intended for the preventive and/or palliative and/or therapeutic treatment of cystic fibrosis is gamma-benzene hexachloride, or of a derivative thereof which is obtainable by chemical substitution, but has retained said capacity of acting as an inducer or stimulator of autophagy maturation.

12. Process for identifying an inducer or stimulator of autophagy maturation, **characterized in that** it comprises:
a) providing a candidate compound,
b) determining whether said candidate compound is capable of phosphorylating a CFTR protein on at least one ERK phosphorylation site, and/or of stimulating such a phosphorylation, and/or of inducing such a phosphorylation,
where a positive determination at said step b) identifies said compound as being an inducer or stimulator of autophagy maturation.

13. Process according to claim 12, **characterized in that** said CFTR protein is a human CFTR protein, and said at least one ERK phosphorylation site is selected from the group consisting of S4, T438, S573, T717, S1235.

14. Process for identifying an inhibitor of autophagy initiation and/or a restorer of autophagy maturation, **characterized in that** it comprises:
a) providing a candidate compound,
b) inducing the initiation of an autophagic process in a eukaryotic CFTR-/- cell,
c) contacting said eukaryotic CFTR-/- cell with said candidate compound,
d) determining whether said candidate compound inhibits or delays the autophagic death of said eukaryotic CFTR-/- cell,
where a positive determination at said step d) identifies said candidate compound as an inhibitor of autophagy initiation and/or as a restorer of autophagy maturation.

15. Process for identifying a compound useful for the manufacture of a drug intended for the preventive and/or palliative and/or therapeutic treatment of a disease or condition involving an excessive production of autophagic structures, **characterized in that** it comprises:
- identifying a compound which induces and/or stimulates autophagy maturation by a process according to any one of claims 12-13, or
- identifying a compound which restores autophagy maturation and/or inhibits autophagy initiation by a process according to claim 14,
where such an identified compound is a compound useful for the manufacture of a drug intended for the preventive and/or palliative and/or therapeutic treatment of a disease or condition involving an excessive production of autophagic structures.

16. Process according to claim 15, **characterized in that** said disease or condition involves an excessive mucus production.

17. Process according to claims 15 and 16, **characterized in that** said disease or condition is cystic fibrosis.

18. Process according to claims 15 and 16, **characterized in that** said disease or condition is asthma, rhinosinusitis, allergy, or an infertility disease or condition.

19. Process according to claims 15 and 16, **characterized in that** said disease or condition is a disease or condition involving at least one viral and/or bacterial and/or fungal infection.

20. Process according to claim 15, **characterized in that** said disease or condition involves the undesired death of a non glandular eukaryotic cell.

21. Process according to claim 20, **characterized in that** said disease or condition is a degenerative disease.

22. **Use of an inducer or stimulator or restorer of autophagy maturation** for the manufacture of a drug intended for the preventive and/or palliative and/or curative treatment of cystic fibrosis.

23. Use according to claim 22, **characterized in that** said inducer or stimulator of autophagy maturation is a CFTR activator.

24. Use according to claim 22 or 23, **characterized in that** said CFTR activator is an activated ERK.

25. Use according to claim 22 or 23, **characterized in that** said CFTR activator is an ERK activator.

26. Use according to any one of claims 22, 23, 25, **characterized in that** said inducer or stimulator of autophagy maturation is gamma-benzene hexachloride, or any derivative thereof, which is obtainable by chemical substitution, but has retained said capacity of acting as an inducer or stimulator of autophagy maturation.

27. Use according to claim 26, **characterized in that** it further comprises using an antioxidant for the manufacture of said drug, wherein said gamma-benzene hexachloride or gamma-benzene hexachloride derivative and said antioxidant are intended for simultaneous, separate or sequential administration.

28. Use of an inhibitor of autophagy initiation for the manufacture of a drug or dietary supplement intended for the preventive and/or palliative and/or curative treatment of cystic fibrosis.

29. Use according to claim 28, **characterized in that** said inhibitor of autophagy initiation comprises at least one aminoacid.

30. Use of gamma-benzene hexachloride, or of a derivative thereof which is obtainable by chemical substitution, but has retained a capacity of acting as an inducer or stimulator of autophagy maturation or as a CFTR activator, for the manufacture of a drug intended for the preventive and/or palliative and/or curative treatment of a disease or condition involving an excessive production of autophagic structures.

31. Use according to claim 30, **characterized in that** it further comprises using an antioxidant for the manufacture of said drug, wherein said gamma-benzene hexachloride or gamma-benzene hexachloride derivative, and said antioxidant are intended for simultaneous, separate or sequential administration.

32. Use according to 30 or 31, **characterized in that** said disease or condition is:
- cystic fibrosis,
- asthma, rhinosinusitis, allergy,
- a disease or condition involving a bacterial and/or viral and/or fungal infection,
- an infertility disease or condition,
- an infertility disease or condition,
- a degenerative disease or condition.

33. Process for identifying an inhibitor of autophagy maturation, **characterized in that** it comprises:
a) providing a CFTR protein or a CFTR protein fragment,
b) determining whether said CFTR protein or CFTR fragment competes with an ERK for binding to or phosphorylating a CFTR protein,
where a positive determination at said step b) identifies said CFTR protein or CFTR fragment as an inhibitor of autophagy maturation.

34. Process for identifying a compound which is an inducer or stimulator of autophagy initiation, **characterized in that** it comprises:
a) providing a candidate compound,
b) contacting said candidate compound with a eukaryotic CFTR-/- cell, and determining whether said candidate compound induces and/or stimulates the autophagic death of said cell,
where a positive determination at said step b) identifies said compound as an inducer or stimulator of autophagy initiation.

35. *In vitro* use of gamma-benzene hexachloride, or of any derivative thereof obtainable by chemical substitution but has retained a capacity of acting as an inducer and/or stimulator of autophagy maturation or as a CFTR activator, to induce and/or stimulate autophagy in a eukaryotic cell.

36. Process for identifying a compound useful for the manufacture of a drug intended for the preventive and/or palliative and/or therapeutic treatment of a disease or condition which involves the undesired survival of a eukaryotic cell that shows an insufficient autophagy initiation, **characterized in that** it comprises:
- identifying a compound which inhibits autophagy maturation, by a process according to claim 33, or
- identifying a compound which induces or stimulates autophagy initiation by a process according to claim 34,
where such an identified compound is a compound useful for the manufacture of a drug intended for the preventive and/or palliative and/or therapeutic treatment of a disease or condition involving the undesired survival of a eukaryotic cell that shows an insufficient autophagy initiation.

37. Process according to claim 36, **characterized in that** said disease or condition which involves an insufficient autophagy is a cancer or tumor condition.

38. Peptide capable of acting as an inhibitor of autophagy maturation by inhibition or prevention of CFTR phosphorylation, **characterized in that** its sequence consists in a sequence selected from SEQ ID NO:1 to SEQ ID NO:56, or a conservative variant sequence thereof, which is obtainable by substitution or deletion, of at least one amino acid, but has retained a capacity of acting as an inhibitor of autophagy maturation.

39. Use of a CFTR inhibitor for the manufacture of an anti-tumour drug intended to induce autophagic cell death of chemo- or radio-resistant cells.

40. Use according to claim 39, **characterized in that** said CFTR inhibitor is a peptide of claim 38.

41. Use according to any one of claims 39-40, **characterized in that** said drug further comprises a chemotherapeutic agent and/or a radiotherapeutic agent for simultaneous, separate or sequential administration.

42. *In vitro* method for the diagnosis and/or prognosis of cystic fibrosis, **characterized in that** it comprises detecting a deficient or insufficient autophagy in a biological sample.

43. *In vitro* method for detecting an autophagy dysfunction in a eukaryotic CFTR-expressing cell, **characterized in that** it comprises detecting whether there is an insufficient phosphorylation of CFTR protein in said eukaryotic cell.

44. *In vitro* method for detecting an autophagy dysfunction in a eukaryotic CFTR-expressing cell, **characterized in that** it comprises detecting whether there is a defective binding of ERK to CFTR in said eukaryotic cell.

45. *In vitro* method for detecting an autophagy dysfunction in a eukaryotic CFTR-expressing cell, **characterized in that** it comprises detecting whether there is a mutation of said CFTR in at least one of its ERK phosphorylation sites, and/or a mutation in at least one site involved in ERK binding to CFTR.

46. *In vitro* method according to claim 48, **characterized in that** said CFTR is a human CFTR, and **in that** it comprises detecting whether there is a mutation of said human CFTR in at least one site selected from:
- any site from R25 to D44,
- sites Q2, R3, S4, P5
- sites L436, G437, T438, P439
- sites L571, D572, S573, P574,
- sites Q715, K716, T717, P718,
- sites S1233, I1234, S1235, P1236,
- sites F1294, I1295, F 1296.

47. *In vitro* method for the diagnosis and/or prognosis of a cystic fibrosis, **characterized in that** it comprises detecting in a biological sample whether there is a deficient content in amino acids, or an abnormal amino acid distribution.

48. *In vitro* method to evaluate the level of autophagic stress that a compound or other external factor may induce in an autophagy-sensitive subject, **characterized in that** it comprises:
a) providing a CFTR-/- cell,
b) *in vitro* exposing said CFTR-/- cell to said compound or factor, and
c) determining the level of autophagic cell death induced by said exposure,
where the level of autophagic cell death thus determined is in positive correlation with the said level of autophagic stress.

49. Kit to assess a risk of autophagic stress for autophagy-sensitive persons, **characterized in that** it comprises at least one Sertoli CFTR-/- cell and/or DCT CFTR-/- cell.

50. Use of a CFTR+/+ Sertoli or CFTR+/+ DCT cell for the detection and/or observation of an autophagic process.

51. *In vitro* method to evaluate the level of deleterious side effects induced by a compound designed for therapy of cystic fibrosis, **characterized in that** it comprises evaluating the level of autophagic cell death induced by *in vitro* exposure of a CFTR-/- cell to said compound.
